(19) Europäisches Patentamt
European Patent Office
Office européen des brevets

(11) Publication number: 0 097 202 B1

# (12) EUROPEAN PATENT SPECIFICATION

(45) Date of publication of patent specification: 19.11.87

(21) Application number: 82105523.3

(22) Date of filing: 23.06.82

(51) Int. Cl.[4]: C 07 D 237/22, C 07 D 237/18, C 07 D 405/12, A 61 K 31/50

## (54) Hydrazinopyridazine compound, process for production thereof, and use thereof as medicament.

(43) Date of publication of application: 04.01.84 Bulletin 84/01

(45) Publication of the grant of the patent: 19.11.87 Bulletin 87/47

(84) Designated Contracting States: AT BE CH DE FR GB IT LI LU NL SE

(56) References cited:
EP-A-0 018 016
EP-A-0 042 593
EP-A-0 092 945
DE-B-2 410 201

Patent Abstracts of Japan, vol. 6, no. 57, 14 April 1982; & JP-A-56 169675

Patent Abstracts of Japan, vol. 6, no. 19, 3 February 1982; & JP-A-56 142272

**The file contains technical information submitted after the application was filed and not included in this specification**

(73) Proprietor: TEIKOKU HORMONE MFG. CO., LTD.
5-1, 2-chome, Akasaka
Minato-ku, Tokyo (JP)

(72) Inventor: Yasuda, Kikuo
19-30, Kakinokidai Midori-ku
Yokohama-shi Kanagawa-ken (JP)
Inventor: Takezaki, Takayuki 5-303, Mukogaoka Century Town
185, Taira Takatsu-ku
Kawasaki-shi Kanagawa-ken (JP)
Inventor: Ohuchi, Rikio
1-2-3-207, Hakusan Tama-ku
Kawasaki-shi Kanagawa-ken (JP)
Inventor: Ohuyabu, Hiroshi
680-117, Ouzenji Tama-ku
Kawasaki-shi Kanagawa-ken (JP)
Inventor: Tanimoto, Yoshitaka
540, Takada-cho Kohoku-ku
Yokohama-shi Kanagawa-ken (JP)
Inventor: Seki, Toshimi
Teikoku Zoki Shataku,1198,Kamiodanaka, Nakahara-ku
Kawasaki-shi, Kanagawa-ken (JP)
Inventor: Yamaguchi, Takashi
302,Tsunashima Mansion, 1-21-17,Tsunashima Nishi
Kohoku-ku, Yokohama-shi, Kanagawa-ken (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 097 202 B1

Courier Press, Leamington Spa, England.

(72) Inventor: Izumi, Akihiro
1993-11, Naruse
Machida-shi Tokyo (JP)
Inventor: Himori, Norio
Teikoku Zoki
Shataku,1198,Kamiodanaka,Nakahara-ku
Kawasaki-shi, Kanagawa-ken (JP)
Inventor: Ishimori, Tsutomu
414,Shinjo Cooporas,390-16,Shinsaku,Takatsu-ku
Kawasaki-shi, Kanagawa-ken (JP)

(74) Representative: Kraus, Walter, Dr. et al
Patentanwälte Kraus, Weisert & Partner
Thomas-Wimmer-Ring 15
D-8000 München 22 (DE)

**Description**

This invention relates to novel hydrazino-pyridazine compounds, a process for producing the aforesaid hydrazinopyridazine compounds or their salts and use of such compounds as antihypertensive agents.

A number of compounds having antihypertensive activity have been proposed up to date. Vasodilators frequently used in the clinical field as antihypertensive agents generally have a definite antihypertensive effect in most hypertensive patients, but their hypotensive action is relatively weak and furthermore they have the defect of inducing tachycardia. β-Adrenoceptor blocking agents have also been increasingly used in the treatment of hypertension for many years.

It is well known that their antihypertensive action is not powerful and sets in slowly. Unlike vasodilators, however, they have the advantage of not causing techycardia. Thus, a sufficient effect cannot always be expected by administering a β-adrenoceptor blocking agent or a vasodilator singly and from this viewpoint, reports have accumulated steadily of the effectiveness of combined therapy with these two types of agents in controlling hypertension. This practice is somewhat troublesome to patients and is not desirable for medication. Accordingly, if a single compound possessing β-adrenoceptor blocking and vasodilating activities is available, it would reduce the patient's compliance and make the treatment of hypertensive patients successful. It has therefore been desired to produce fascinating antihypertensive agents which show rapid and accurate antihypertensive activity without causing reflex tachycardia. Recently, some anti-hypertensive agents having both β-adrenoceptor blocking and vasodilating activities were proposed (see DE—A—2 527 066 and 2 556 918), although these patent documents give only a small amount of data concerning their β-adrenoceptor blocking and vasodilating activities.

The present inventors proposed a series of hydrazinopyradinol derivatives as antihypertensive agents free from the aforesaid defects in JP—A—142272/1981 and 169675/1981. On further investigations, the present inventors have discovered the compounds of formula (I) given above. The hydrazinopyridazine compounds of formula (I) described in this invention have not only β-adrenoceptor blocking action but also excellent vasodilating action as demonstrated by pharmacological data to be given hereinbelow (Table 1). In view of the pharmacological feature and activity of these compounds, they seem to be very suitable and promising as antihypertensive agents.

EP—A—92 945 describes certain hydrazinopyridazine derivatives possessing an antihypertensive activity. Compounds comprising a hydrazinopyridazine group showing an antihypertensive activity are known from EP—A—18 016. Compounds comprising the 1-amino-3-aryloxy-propan-2-ol residue showing a β-adrenergic blocking activity are known from EP—A—42 593.

The present invention provides new hydrazinopyridazine compounds represented by the formula

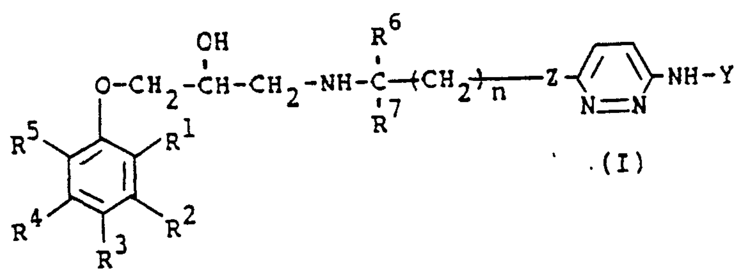

(I)

wherein

$R^6$ and $R^4$, independently from each other, represent a hydrogen atom or a methyl group,

Z represents

$$-O-,\ -S-,\ \text{or}\ -\underset{\displaystyle R^8}{\underset{|}{N}}-$$

in which $R^8$ represents a hydrogen atom or a $C_1$—$C_5$ alkyl group;

Y represents

$$-NH_2,\ -NH-COOC_2H_5,\ \text{or}\ -N{=}C\begin{matrix}CH_3\\ CH_3\end{matrix}\ ;$$

n is 1 or 2; and

(a) when Z represents —O—,

$R^1$ represents a hydrogen atom, a $C_1$—$C_5$ alkyl group substituted by a $C_1$—$C_5$ alkoxy, $C_1$—$C_5$ alkylthio, $C_2$—$C_5$ alkanoylamino, allyloxy or tetrahydrofurfuryloxy group, a $C_1$—$C_5$ alkoxy group substituted by a 2-furyl, phenyl or $C_1$—$C_5$ alkoxy group,

$R^2$ and $R^3$, independently from each other, represent a hydrogen atom or a $C_1$—$C_5$ alkyl group substituted by a $C_1$—$C_5$ alkoxy group,

with proviso that $R^1$, $R^2$ and $R^3$ are not simultaneously hydrogen atoms, and that when $R^3$ represents a $C_1$—$C_5$ alkyl group substituted by a $C_1$—$C_5$ alkoxy group, $R^1$ may represent a halogen atom, and

$R^4$ and $R^5$ represent a hydrogen atom; and

(b) when Z represents

—S— or 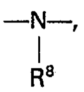

$R^1$ represents a methoxyethyl or $C_1$—$C_5$ alkyl group, or a halogen atom,

$R^2$ and $R^4$, independently from each other, represent a halogen atom or a methyl group,

$R^3$ and $R^5$ represent a hydrogen atom, or its salt.

Since the asterisked carbon atom in the compound of formula (I) shown below is an asymmetric carbon atom, it is evident that it can exist as an optically active or racemic form.

It should be understood therefore that the compounds of formula (I) embrace both of these optically active and racemic forms.

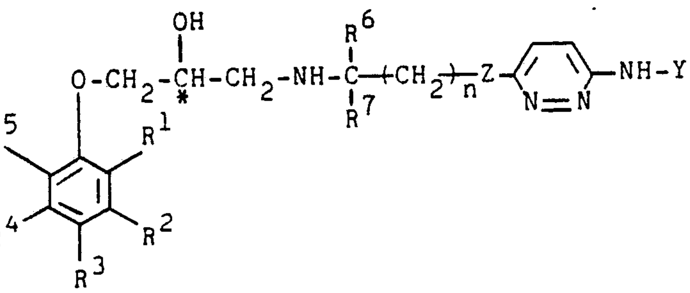

wherein $R^1$, $R^2$, $R^3$, $R^4$, $R^5$, $R^6$, $R^7$, Z, Y and n are as defined hereinabove.

The $C_1$—$C_5$ alkyl group, as used herein, may be linear or branched, and includes, for example, methyl, ethyl, n- or iso-propyl, and n-, iso-, sec- or tert-butyl groups. Methyl and ethyl groups are suitable.

The $C_1$—$C_5$ alkoxy group includes, for example, methoxy, ethoxy, n-propoxy and isopropoxy groups.

Examples of the $C_1$—$C_5$ alkylthio group are methylthio and ethylthio groups.

An acetylamino group is an example of the $C_2$—$C_5$ alkanoylamino group.

The halogen atom means fluorine, chlorine, bromine and iodine atoms. Fluorine, chlorine and bromine atoms are preferred.

Examples of the $C_1$—$C_5$ alkyl group substituted by a $C_1$—$C_5$ alkoxy, $C_1$—$C_5$ alkylthio, $C_2$—$C_5$ alkanoylamino, allyloxy or tetrahydrofurfuryloxy group are acetylaminomethyl, 2-acetylaminoethyl, ethoxymethyl, 2-methoxyethyl, 2-ethoxyethyl, 3-methoxypropyl ethylthiomethyl, 2-methylthioethyl, 2-tetrahydrofurfuryloxyethyl and 2-allyloxyethyl groups.

Examples of a $C_1$—$C_5$ alkoxy group substituted by a 2-furyl, phenyl or $C_1$—$C_5$ alkoxy group are furfuryloxy, benzyloxy, phenethyloxy, methoxymethoxy, 2-methoxyethoxy and 2-ethoxyethoxy groups.

Preferred examples of $R^1$ in formula (I) are a chlorine atom and methyl, ethyl, methoxymethyl and 2-methoxyethyl groups, and the chlorine atom and methyl groups are especially preferred. Examples of $R^2$ are a chlorine atom and a methyl group, and the methyl group is especially preferred. A chlorine atom and a methyl group are preferred as $R^4$.

A preferred group of compounds of formula (I) includes those compounds of formula (I) in which Z is an oxygen atom and/or $R^6$ and $R^7$ are both methyl groups, and/or n is 1, above all compounds of the following formula (I-a)

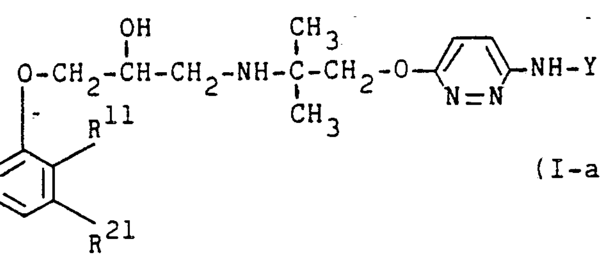

(I-a)

wherein

$R^{11}$ represents a methoxymethyl or 2-methoxyethyl group,

$R^{21}$ represents a hydrogen atom,

$R^{41}$ represents a hydrogen atom, and
Y is as defined above.
In formula (I-a), Y is preferably $-NH_2$.

Another preferred group of the hydrazinopyridazine compounds of formula (I) includes those of formula (I) in which $R^1$ represents a chlorine atom or a methyl group, and $R^2$, $R^3$, $R^4$ and $R^5$ are hydrogen atoms.

Typical examples of the hydrazinopyridazine compounds of formula (I) provided by this invention include the following compounds in addition to those given in Examples.

1-[2-(2-methoxyethyl)phenoxy]-3-[1-methyl-2-(3-hydrazino-6-pyridazinyloxy)ethylamino]-2-propanol,

1-[2-(2-methoxyethoxy)phenoxy]-3-[1-methyl-2-(3-hydrazino-6-pyridazinyloxy)ethylamino]-2-propanol,

1-(2-ethoxymethoxyphenoxy)-3-[1,1-dimethyl-2-(3-hydrazino-6-pyridazinyloxy)ethylamino]-2-propanol,

1-[2-(2-methoxyethyl)phenoxy]-3-[2-(3-hydrazino-6-pyridazinyloxy)ethylamino]-2-propanol,

1-[2-(3-methoxypropyl)phenoxy]-3-[1,1-dimethyl-2-(3-hydrazino-6-pyridazinyloxy)ethylamino]-2-propanol,

1-[2-(2-ethoxyethyl)phenoxy]-3-[1,1-dimethyl-2-(3-hydrazino-6-pyridazinyloxy)ethylamino]-2-propanol, and

1-[2-(2-methylthioethyl)phenoxy]-3-[1,1-dimethyl-2-(3-hydrazino-6-pyridazinyloxy)ethylamino]-2-propanol.

According to this invention, there is provided an acid addition salt of the hydrazinopyridazine compound of formula (I). Examples of the acid addition salt of the compound of formula (I) are salts of inorganic acids such as hydrochloric acid, hydrobromic acid, sulfuric acid, nitric acid and phosphoric acid, and salts of organic acids such as acetic acid, propionic acid, citric acid, lactic acid and tartaric acid. Those acid addition salts which are pharmaceutically acceptable are advantageous.

According to this invention, the hydrazinopyridazine compound of formula (I) or its salt can be produced by reacting a compound of the following formula (II)

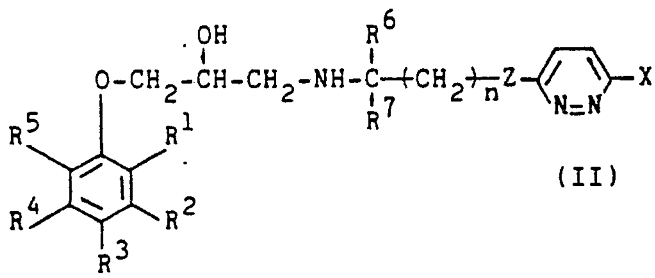

(II)

wherein

$R^1$, $R^2$, $R^3$, $R^4$, $R^5$, $R^6$, $R^7$, Z and n are as defined above, and X represents a halogen atom, preferably a chlorine, bromine or iodine atom, especially preferably a chlorine atom, with (a) hydrazine or hydrazine hydrate when Z in formula (II) represents —O— or —S—, or (b) ethyl carbazinate when Z in formula (II) represents

$$\underset{\displaystyle R^8}{\overset{\displaystyle -N-}{|}};$$

hydrolyzing the resulting compound of formula (I) in which Y is $-NH-COOC_2H_5$; then as necessary, reacting the resulting compound of formula (I) in which Y is $-NH_2$ with acetone; and as necessary converting the resulting compound of formula (I) to a salt.

The reaction of the compound of formula (II) in which Z is —O— or —S— [to be referred to hereinafter as the compound of formula (II-a)] with hydrazine or hydrazine hydrate, preferably the latter, can be carried out by contacting the reactants in the absence of a solvent or in the presence of an inert solvent. Examples of inert solvents which can be used include water; alcohols such as methanol, ethanol, propanol and isopropanol; ethers such as dioxane and tetrahydrofuran; organic bases such as pyridine and collidine; and aromatic hydrocarbons such as benzene, toluene and xylene.

The reaction temperature is not critical, and can be varied widely depending upon the type of the starting material of formula (II-a). Generally, it is advantageous that the reaction is carried out at a temperature of at least 20°C, preferably about 50°C to the refluxing temperature of the reaction mixture.

The amount of hydrazine or its hydrate based on the compound of formula (II-a) is neither critical, and can be varied widely depending upon the type of the compound of formula (II-a) or the reaction conditions. Generally, it is at least l2 moles, preferably about 10 to about 200 moles, more preferably about 50 to about 100 moles, per mole of the compound of formula (II-a).

The reaction time varies depending upon the type of the compound of formula (II-a) or the reaction temperature. Usually, the reaction can be terminated in about 0.5 to about 10 hours.

The above reaction may be carried out in the presence of a suitable base. Examples of bases which can be used include alkali metal carbonates such as potassium carbonate and sodium carbonate; alkali metal hydroxides such as potassium hydroxide and sodium hydroxide; and organic bases such as pyrrolidone, piperazine, piperidine, morpholine and 4-dimethyl-aminopyridine. The base can be used in an amount of about 1 to about 100 equivalents, preferably about 1 to about 10 equivalents, per mole of the compound of formula (II-a). At this time, hydrazine or its hydrate may be used in a large excess so that the excess of hydrazine or its hydrate acts as a base.

By the above reaction, the compound of formula (I) in which Y is $-NH_2$, that is, a hydrazine derivative of formula (I-b) below, is obtained.

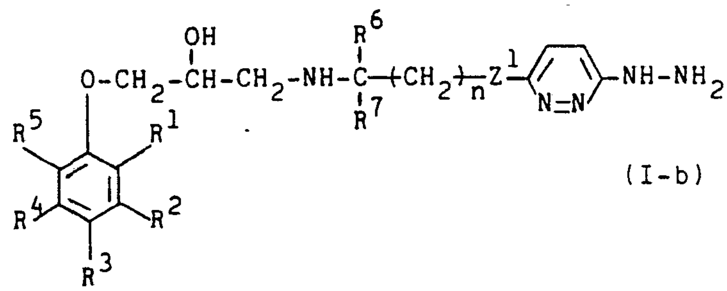

(I-b)

wherein $R^1$, $R^2$, $R^3$, $R^4$, $R^5$, $R^6$, $R^7$ and n are as defined hereinabove, and $Z^1$ represents —O— or —S—.

On the other hand, the reaction of the compound of formula (II) in which Z represents

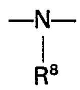

[to be referred to hereinafter as the compound of formula (II-b)] with ethyl carbazinate can be carried out in the absence of a solvent or in the presence of an inert solvent. Examples of the inert solvent are alcohols such as propanol and butanol; ethers such as dioxane and tetrahydrofuran; aromatic hydrocarbons such as toluene, xylene and tetralin; and hydrocarbons such as cyclohexane and decalin.

The reaction temperature is not critical and can be widely varied depending upon the type of the starting material of formula (II-b). Generally, it is about 80 to about 160°C, preferably about 120 to about 150°C.

The amount of ethyl carbazinate is not particularly restricted. Conveniently, it is generally about 1 to about 20 moles, preferably about 2 to about 10 moles, per mole of the compound of formula (II-b).

The reaction of the compound of formula (II-b) with ethyl carbazinate can be terminated in about 0.5 to about 3 hours under these conditions.

By the above reaction, the compound of formula (I) in which Y is $-NH-COOC_2H_5$, that is, a compound of the following formula (I-c), can be obtained.

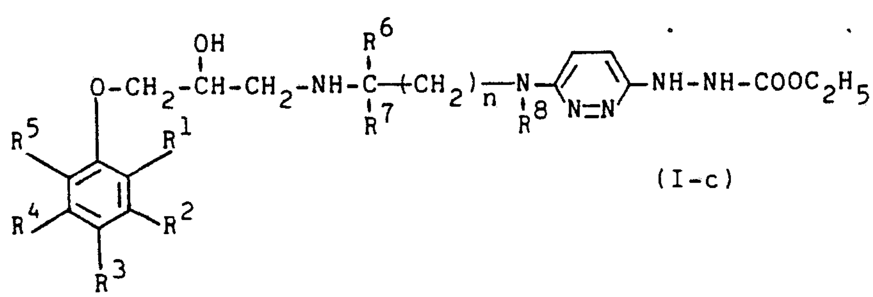

(I-c)

wherein $R^1$, $R^2$, $R^3$, $R^4$, $R^5$, $R^6$, $R^7$, $R^8$ and n are as defined hereinabove.

As required, the compound of formula (I-c) may be hydrolyzed to split off the carboethoxy group from the carboethoxyhydrazino group moiety to give a compound of the following formula

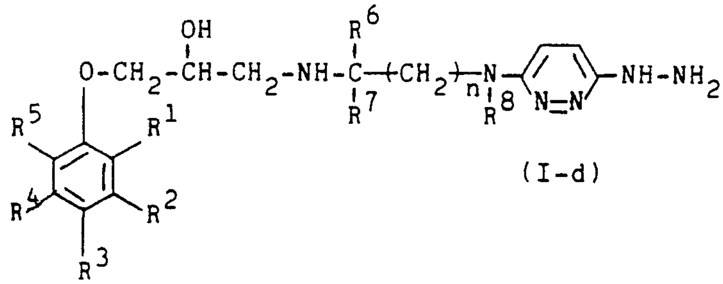

(I-d)

wherein all symbols are as defined.

Hydrolysis of the compound of formula (I-c) can be carried out by a method known *per se*, for example at room temperature to the refluxing temperature of the reaction mixture using an acid such as hydrochloric acid and hydrobromic acid.

As required, the resulting compound of formula (I-b) or (I-d) may be reacted with acetone to give a compound of formula (I) in which Y is

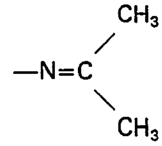 .

Conversion of the compound of formula (I-b) or (I-d) into a hydrazone compound by using acetone can be carried out by contacting the compound of formula (I-b) or (I-d) with acetone in the absence of a solvent or in the presence of an inert solvent. Examples of the inert solvent include water; alcohols such as methanol, ethanol and propanol; ethers such as diethyl ether, dioxane and tetrahydrofuran; aromatic hydrocarbons such as benzene, toluene and xylene; and halogenated hydrocarbons such as dichloromethane, chloroform and tetrachloroethane. The reaction proceeds sufficiently smoothly even at room temperature. If desired, the reaction may be carried out by heating the reactants to the refluxing temperature of the reaction mixture, preferably to a temperature below about 100°C.

The amount of acetone based on the compound of formula (I-b) or (I-d) is not critical, and can be varied widely depending upon the type of the compound of formula (I-b) or (I-d). Generally, it is advantageous to use acetone in an amount of at least 1 mole, preferably about 2 to about 100 moles, especially preferably about 10 to about 50 moles, per mole of the compound of formula (I-b) or (I-d).

This reaction proceeds very rapidly and almost quantitatively, and ends in about 10 to about 60 minutes.

Thus, the hydrazinopyridazine compound of formula (I) is obtained. It can be separated from the reaction mixture and/or can be purified by various known procedures such as extraction, column chromatography, thin-layer chromatography and recrystallization.

When both of $R^6$ and $R^7$ in the compound of formula (I) are methyl groups or hydrogen atoms, the racemic mixture can, as necessary, be separated into optically active forms by, for example, going through a diastereomer salt of the compound of formula (I), for example its salt with tartaric acid, malic acid, camphoric acid, camphor-sulfonic acid, etc.

The aforesaid optical resolution may be applied to the starting compound of formula (II), and in this case, too, the aforesaid method of optical resolution can be used. By introducing the hydrazino group into the resulting optically active starting compound, it can be converted to an optically active compound of formula (I).

If further required, the hydrazinopyridazine compound of formula (I) can be converted to its salt by treating it in a customary manner with an inorganic acid such as hydrochloric acid, hydrobromic acid, sulfuric acid, nitric acid or phosphoric acid, or an organic acid such as acetic acid, propionic acid, citric acid, lactic acid or tartaric acid.

The compounds of formula (II) used as starting materials in the above process of this invention are novel.

Typical examples include the following compounds in addition to those given in Examples.

1-[2-(2-methoxyethyl)phenoxy]-3-[1,1-dimethyl-2-(3-bromo-6-pyridazinyloxy)ethylamino]-2-propanol,
1-(2-ethoxymethylphenoxy)-3-[1,1-dimethyl-2-(3-chloro-6-pyridazinyloxy)ethylamino]-2-propanol,
1-[2-(2-methoxyethyl)phenoxy]-3-[1-methyl-2-(3-chloro-6-pyridazinyloxy)ethylamino]-2-propanol, and
1-[2-(2-methoxyethyl)phenoxy]-3-[1,1-dimethyl-2-(3-bromo-6-pyridazinylthio)ethylamino]-2-propanol.

These compounds of formula (II) can be synthesized by the process shown in Scheme A when Z is —O— or —S—, and by the process shown in Scheme B when Z is

$$-\underset{\substack{|\\R^8}}{H}-.$$

Scheme A

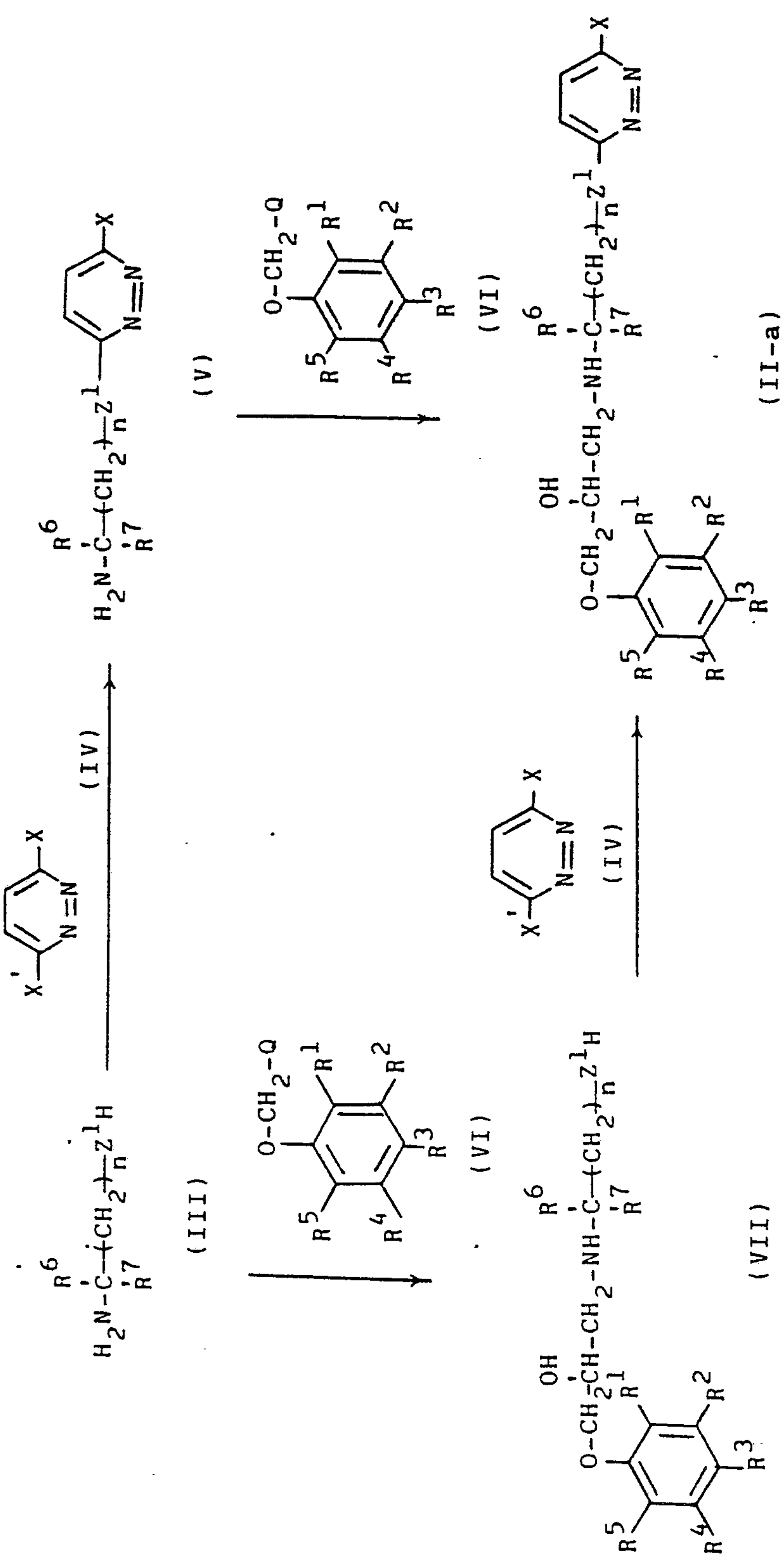
(III) (IV) (V) (VI) (VII) (VI) (IV) (II-a)

In Scheme A, $R^1$, $R^2$, $R^3$, $R^4$, $R^5$, $R^6$, $R^7$, $Z^1$, n and X are as defined hereinabove; X′ represent a halogen atom which is identical with or different from X; and Q represents

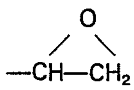 or 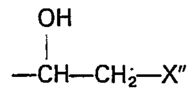

in which X″ represents a halogen atom.

According to a first embodiment in Scheme A, the compound of formula (IV) known *per se* is reacted with the compound of formula (III). This reaction can be carried out by using the compound (III) in the form of an alkoxide or alkylsulfide corresponding to formula (III) in which $—Z^1H$ changes to $—Z^1M$ wherein M is an alkali metal (for example, a sodium alkoxide, a potassium alkoxide or a sodium alkylsulfide), or using the compound (III) in the presence of a base such as sodium hydride, sodium amide, sodium hydroxide, potassium hydroxide or lithium hydroxide, and contacting it with the compound (IV). The reaction can be carried out in the absence of a solvent, or preferably in the presence of an inert solvent, for example an aromatic hydrocarbon (e.g., benzene, toluene or xylene), an ether (e.g., dioxane, tetrahydrofuran, diethyl ether or dimethoxyethane), an amide such as dimethylformamide, or an organic base (e.g., pyridine, triethylamine or dimethylaniline) at a temperature of from 0°C to the refluxing temperature of the reaction mixture, preferably from room temperature to the refluxing temperature of the reaction mixture.

The proportions of the compounds of formulae (IV) and (III) are not particularly restricted. Advantageously, at least 1 mole, preferably about 1 to about 2 moles, of the compound (III) is used per mole of the compound (IV). Under these reaction conditions, the reaction can be terminated in about 0.5 to 5 hours.

Thus, the compound of formula (V) can be obtained in good yields. Typical examples of the compound (V) are 3-(2-amino-2-methylpropoxy)-6-chloropyridazine, 3-(2-amino-2-methylpropylthio)-6-chloropyridazine, 3-(2-aminopropoxy)-6-chloropyridazine, 3-(2-aminopropylthio)-6-chloropyridazine, 3-(2-aminoethoxy)-6-chloropyridazine, and 3-(2-amino-2-methylpropoxy)-6-bromopyridazine.

The compound of formula (II) can be produced by reacting the compound of formula (V) with the compound of formula (VI). This reaction can be easily carried out by contacting the compound (V) and the compound (VI) in the presence or absence of a solvent, preferably in the presence of an inert organic solvent. The reaction temperature is not critical, and can be properly selected. Generally, it is about 0°C to about 200°C, preferably from room temperature to about 100°C. In the present reaction, about 1 to about 2 moles of the compound (V) can be used per mole of the compound (VI).

The reaction time can be properly varied depending upon the type of the reactants, the reaction temperature, etc., and is generally from about 1 to 5 hours.

Examples of inert solvents which can be used in the aforesaid reaction include water; lower alcohols such as methanol, ethanol and isopropanol; aromatic hydrocarbons such as benzene, xylene and toluene; and halogenated hydrocarbons such as chloroform, dichloromethane, dichloroethane, trichloroethane and carbon tetrachloride.

The compound (VI) in which Q is

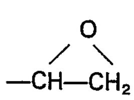

in the first embodiment described above is obtained, for example, by reacting a compound of the following formula (VIII)

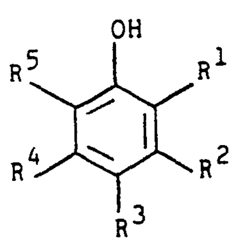

(VIII)

wherein $R^1$, $R^2$, $R^3$, $R^4$ and $R^5$ are as defined hereinabove, with an epihalohydrin. By treating the resulting compound (VI) in which Q is

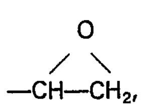,

that is, a compound of the following formula (VI-a)

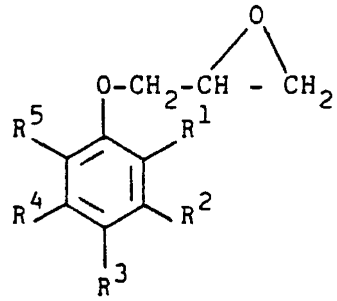

(VI-a)

wherein $R^1$, $R^2$, $R^3$, $R^4$ and $R^5$ are as defined above, with a hydrohalic acid (HX'') to cleave the epoxy group, a compound of formula (VI) in which Q represents

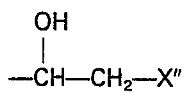

can be produced.

The reaction of the compound (VII) with an epihalohydrin can be carried out in a customary manner, for example at room temperature to about 100°C, preferably in the absence of a solvent or in the presence of water or an alcohol such as methanol, ethanol or propanol, for about 1 to about 5 hours under alkaline conditions. This reaction can give the desired product (VI-a) in good yields.

The reaction of cleaving the epoxy group of the compound (VI-a) with a hydrohalic acid such as hydrochloric acid or hydrobromic acid can be carried out in a customary manner, for example at 0°C to the refluxing temperature of the reaction mixture, preferably in the presence of an inert organic solvent such as chloroform, ethanol, benzene or dioxane, for a period of about 0.5 to about 5 hours.

According to a second embodiment in Scheme A, the compound of formula (III) is reacted with the compound of formula (VI). This reaction can be carried out in the same way as described above with regard to the reaction of the compounds (V) and (VI).

Thus, the compound of formula (VII) is obtained.

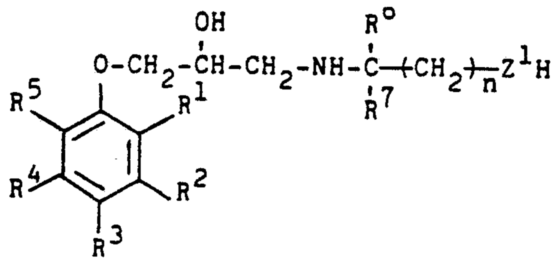

(VII)

wherein all symbols are as defined above.

This compound (VII) can be converted to the compound of formula (II) by reacting it with the compound (IV).

The reaction of the compound (VII) with the compound (IV) can be carried out in the same way as described hereinabove in regard to the reaction of the compound (III) with the compound (IV) in the first embodiment.

The compound of formula (II) produced by the first or second embodiment is usually separated from the reaction mixture by methods known *per se*, and then used for reaction with hydrazine or its hydrate.

Scheme B

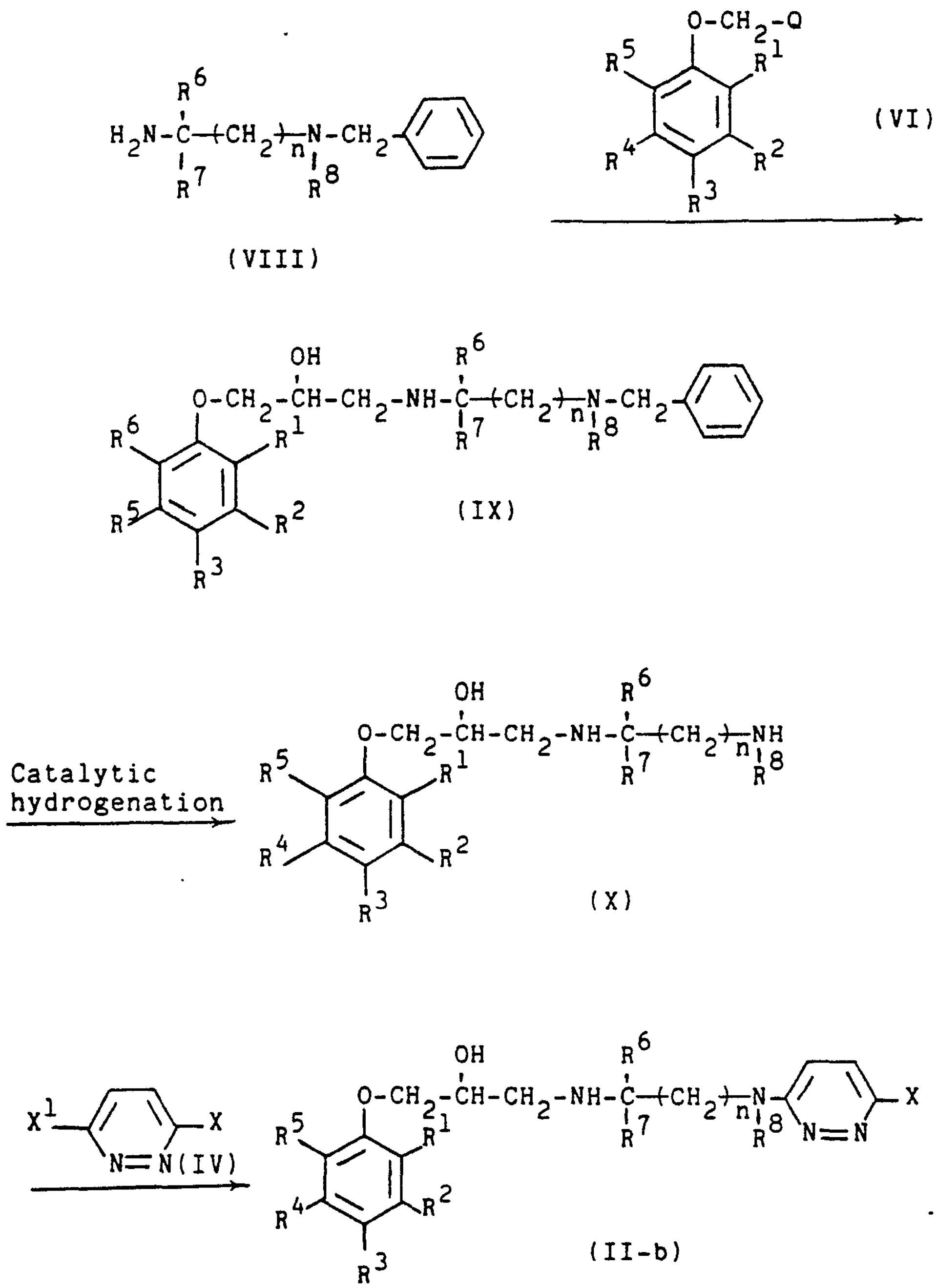


(VIII) (VI) (IX) (X) (IV) (II-b)

In Scheme B, all symbols are as defined hereinabove.

In Scheme B, the reaction of the compound of formula (VIII) with the compound of formula (VI) can be carried out under the same conditions as described above with regard to the reaction of the compound (III) with the compound (VI).

The resulting compound of formula (IX) is then catalytically hydrogenated to form the compound of formula (X). Hydrogenation can be performed by contacting the compound (IX) with a hydrogen gas at atmospheric pressure to an elevated pressure of up to about 100 atmospheres in the presence of a hydrogenation catalyst such as palladium-carbon palladium black, platinum black or Raney nickel in a suitable inert solvent, for example an alcohol such as methanol, ethanol or propanol, an ether such as dioxane or tetrahydrofuran, a halogenated hydrocarbon such as chloroform or dichloromethane, or an acid such as dilute hydrochloric acid or acetic acid.

By the hydrogenation, the benzyl group is split off from the compound (IX), and the compound (X) results.

The compound (X) is then reacted with the compound of formula (IV). This reaction can be carried out in the absence of a solvent or in the presence of an inert solvent, for example an alcohol such as propanol or butanol, an ether such as dioxane or tetrahydrofuran, or an aromatic hydrocarbon such as toluene or xylene at a temperature of generally about 0°C to the refluxing temperature of generally about 0°C to the refluxing temperature of the reaction mixture, preferably at a temperature of from 50 to 120°C. The proportions of the compounds (X) and (IV) are not critical, and can be widely changed according to the types of these compounds, etc. Conveniently, the compound (IV) is used in an amount of generally at least 1 mole, preferably about 2 to about 4 moles, per mole of the compound (X).

Thus, the compound of formula (II-b) can be obtained. This compound can usually be separated from the reaction mixture by methods known *per se,* and then used for reaction with ethyl carbazinate.

To evaluate the activities of the compounds of formula (I) provided by this invention, the following experiments were performed.

The following compounds were submitted to animal experiments.

L: 1-(2-methoxymethylphenoxy)-3-[1,1-dimethyl-2-(3-hydrazino-6-pyridazinyloxy)ethylamino]-2-propanol

M: 1-(2-ethoxymethylphenoxy)-3-[1,1-dimethyl-2-(3-hydrazino-6-pyridazinyloxy)ethylamino]-2-propanol

N: 1-[2-(2-methoxyethyl)phenoxy]-3-[1,1-dimethyl-2-(3-hydrazino-6-pyridazinyloxy)ethylamino]-2-propanol

O: 1-[2-(2-ethoxyethyl)phenoxy]-3-[1,1-dimethyl-2-(3-hydrazino-6-pyridazinyloxy)ethylamino]-2-propanol

Q: 1-(2-methylphenoxy)-3-[1,1-dimethyl-2-(3-hydrazino-6-pyridazinylthio)ethylamino]-2-propanol

Testing methods

Experiments were done using male Wistar rats weighing between 300 and 400 g, anesthetized with sodium pentobarbital (60 mg/kg i.p.). The blood pressure was directly measured by connecting a cannula inserted in the femoral artery to a pressure transducer and the heart rate was calculated from the pulse waves of the blood pressure tracings. Each value shown here was obtained from three animals. The test compounds were individually dissolved in physiological saline solution or a 0.01N aqueous solution of hydrochloric acid.

(1) Measurement of β-adrenoceptor blocking activity

A single intravenous (i.v.) injection of isoproterenol (0.1 μg/kg) produced an increase in heart rate by approximately 90 beats/min. The increased heart rate before injection of the test compound was represented by $H_1$. Three minutes after i.v. injection of the test compound, isoproterenol (0.1 μg/kg i.v.) was again administered and then the change in heart rate was measured. The maximum increase in heart rate after injection of the test compound was designated as $H_2$. From these values, the percent inhibition of the change in heart rate was calculated in accordance with the following equation.

Percent inhibition of the heart rate

$$= 100 - \frac{H_2}{H_1} \times 100$$

By repeating the procedure mentioned above at various doses of the test compounds, dose-response curves were prepared. From these curves, their respective $ID_{50}$ values (doses of test compounds required for 50% inhibition of responses to isoproterenol) were determined. The results are shown in Table 1.

(2) Measurement of vasodilating activity (Hypotensive activity)

Each test compound dissolved in physiological saline solution or 0.01N hydrochloric acid was intravenously injected into rats in a fixed dose of 1 mg/kg and the blood pressure was continuously monitored for 40 minutes. The maximum fall in blood pressure during this period was determined and taken as the hypotensive activity of each compound. The results are shown in Table 1.

TABLE 1

| Compound | β-adrenoceptor blocking action $ID_{50}$ (mg/kg i.v.) | Hypotensive action |
|---|---|---|
| L | 0.03 | +++ |
| M | 0.06 | ++ |
| N | 0.08 | +++ |
| O | 0.16 | ++ |
| Q | 0.04 | ++ |

In Table 1, the marks, ++ and +++, represent a fall in blood pressure of 0,033 to 0.045 bar (25 to 34 mmHg), and 0,0366 bar (35 mmHg) or more, respectively.

Thus, the hydrazinopyridazine compounds of formula (I) provided by this invention are novel β-adrenoceptor blocking agents. Accordingly, they can be used as antihypertensive agents for man and warm-blooded animals and, for this purpose, administered orally or parenterally (e.g., intramuscularly, intravenously, subcutaneously, intrarectally, sublingually, etc.).

When used as antihypertensive agents, the compounds (I) of this invention can be formulated into various forms suitable for oral or parenteral administration. For example, they can be formed into drugs by using nontoxic additives usually employed in this type of drugs, such as vehicles, binders, lubricants, disintegrants, antiseptics, isotonic agents, stabilizers, dispersants, antioxidants, coloring agents, flavoring agents, and buffers.

Depending upon the intended use, these drugs may take various forms, for example solids (e.g., tablets, hard capsules, soft capsules, granules, powders, pellets, pills and trouches), semisolids (e.g. suppositories), and liquids (injectable preparations, emulsions, suspensions and syrups).

Examples of the nontoxic additives mentioned above include starch, gelatin, glucose, lactose, fructose, maltose, magnesium carbonate, talc, magnesium stearate, methyl cellulose, carboxymethyl cellulose or its salt, gum arabic, polyethylene glycol, alkyl p-hydroxybenzoates, syrup, ethanol, propylene glycol, Vaseline, carbowax, glycerol, sodium chloride, sodium sulfite, sodium phosphate and citric acid.

The above drugs may also contain therapeutically useful other drugs.

The content of the compound (I) of this invention in the drugs may vary depending upon its form. Generally, it is preferably 5 to 100% by weight in the case of solid and semisolid drugs, and 0.1 to 10% by weight in the case of liquid drugs.

The dose of the compound (I) of this invention can be varied widely depending upon the type of the subject, the severity of the subject's condition, a physician's diagnosis, etc. Generally, it is 0.02 to 30 mg/kg, preferably 0.05 to 10 mg/kg, per day. The dose may be below or above the specified limit depending upon the severity of the condition of the patient, and the diagnosis of a physician. The above daily dose may be given at a time or in two or more divided portions.

The following Examples illustrate the present invention more specifically.

NMR spectra were measured with Hitachi R—20A (60 MHz) using tetramethylsilane as an internal standard in $CDCl_3$ and $CD_3OD$, and sodium 2-dimethyl-2-silapentane-5-sulfonate in $D_2O$.

Example 1

(a) To a suspension of 1.5 g of 61% sodium hydride in 20 ml of benzene was added dropwise at room temperature 2.7 g of 2-amino-2-methylpropanol, and then a solution of 4.5 g of 3,6-dichloropyridazine in 20 ml of benzene was added. The mixture was refluxed for one hour. The reaction mixture was cooled, washed with water, and dried over magnesium sulfate. The solvent was evaporated to give 4.33 g of 3-(2-amino-2-methylpropoxy)-6-chloropyridazine.

NMR($CDCl_3$) δ: 1.22 (6H, s), 1.49 (2H, s), 4.23 (2H, s), 7.03 (1H, d, J=9Hz), 7.42 (1H, d, J=9Hz).

(b) A mixture of 4.83 g of 3-(2-amino-2-methylpropoxy)-6-chloropyridazine obtained in (a) above, 5.0 g of 1-(2-(2-methoxyethyl)phenoxy)-2,3-epoxypropane and 200 ml of ethanol was refluxed for 4 hours, and the solvent was evaporated under reduced pressure. A solution of the residue in benzene was extracted with 1N hydrochloric acid. The aqueous layer was extracted with chloroform, washed successively with 5% sodium carbonate and water, and the organic layer was dried over magnesium sulfate. The solvent was evaporated to give 3.8 g 1 - (2 - (2 - methoxyethyl)phenoxy) - 3 - (1,1 - dimethyl - 2 - (3 - chloro - 6 - pyridazinyloxy)ethylamino) - 2 - propanol.

NMR($CDCl_3$) δ: 1.21 (6H, s), 2.30—2.70 (2H, m), 2.70—3.05 (4H, m), 3.30 (3H, s), 3.57 (2H, t, J=6Hz), 4.00 (3H, broad s), 4.33 (2H, s), 6.70—7.24 (4H, m), 6.93 (1H, d, J=9Hz), 7.32 (1H, d, J=9Hz.

(c) A mixture of 1.8 g of 1 - (2 - (2 - methoxyethyl)phenoxy) - 3 - (1,1 - dimethyl - 2 - (3 - chloro - 6 - pyridazinyloxy)ethylamino) - 2 - propanol obtained in (b) above, 15 ml of hydrazine hydrate and 30 ml of ethanol was refluxed overnight with stirring. The solvent was removed under reduced pressure. A solution of the residue in 90 ml of acetone was refluxed for 15 minutes. After evaporating the solvent, a solution of the oily residue in chloroform was washed successively with 5% sodium carbonate and an aqueous solution of sodium chloride and dried over magnesium sulfate. The solvent was evaporated and the crude product was purified by thin-layer chromatography (silica gel (Merck $GF_{254}$); chloroform/methanol=9/1) to give 219 mg of 1 - (2 - (2 - methoxyethyl)phenoxy) - 3 - (1,1 - dimethyl - 2 - (3 - isopropylidene-hydrazino - 6 - pyridazinyloxy)ethylamino) - 2 - propanol as a pale yellow oil.

NMR($CD_3OD$) δ: 1.25 (6H, s), 1.94, 2.00 (6H, double s), 2.50—3.10 (4H, m), 3.29 (3H, s), 3.52 (2H, t, J=6Hz), 3.98 (3H s), 4.20 (2H, s), 6.98 (1H, d, J=9Hz), 7.46 (1H, d, J=9Hz), 6.66—7.30 (4H, m).

(d) A mixture of 2.0 g of 1 - (2 - (2 - methoxyethyl)phenoxy) - 3 - (1,1 - dimethyl - 2 - (3 - chloro - 6 - pyridazinyloxy)ethylamino) - 2 - propanol obtained in (b) above, 20 ml of hydrazine hydrate and 40 ml of ethanol was refluxed overnight. After removing the solvent under reduced pressure, a solution of the residue in chloroform was washed with water, dried over magnesium sulfate, and concentrated to dryness. To a solution of the residue in ethanol was added hydrogen chloride ether solution. The solvent was evaporated under reduced pressure. A solution of the crude product in 2 ml of ethanol was added dropwise to 20 ml of ether. The resulting precipitate was separated by decantation to give 1.0 g of 1 - (2 - (2 - methoxyethyl)phenoxy) - 3 - (1,1 - dimethyl - 2 - (3 - hydrazino - 6 - pyridazinyloxy)ethylamino) - 2 - propanol hydrochloride having a melting point of 173.3 to 174.1°C.

NMR($D_2O$) δ: 1.58 (6H, s), 2.82 (2H, t, J=6Hz), 3.23—3.49 (2H, m), 3.33 (3H, s), 3.65 (2H, t, J=7Hz), 3.99—4.35 (3H, m), 4.42 (2H, s), 6.80—7.45 (4H, m), 7.28 (2H, s).

In the same way as in Example 1, 3-(2-amino-2-methylpropoxy)-6-chloropyridazine was reacted with the corresponding epoxide and then the product was converted to the corresponding hydrazine or hydrazone compound. Thus, the compounds shown in Examples 2 to 10 below were obtained.

## Example 2

(a) 1-(2-Methoxymethylphenoxy)-3-[1,1-dimethyl-2-(3-chloro-6-pyridazinyloxy)ethylamino]-2-propanol.

NMR ($CDCl_3$) δ: 1.20 (6H, s), 2.67—2.90 (2H, m), 2.95 (2H, s), 3.33 (3H, s), 3.98 (3H, broad s), 4.31 (2H, s), 4.43 (2H, s), 6.70—7.40 (4H, m), 6.90 (1H, d, J=9Hz), 7.30 (1H, d, J=9Hz).

(b) 1-(2-Methoxymethylphenoxy)-3-[1,1-dimethyl-2-(3-hydrazino-6-pyridazinyloxy)ethylamino]-2-propanol hydrochloride.

Melting point: 175.3—179.7°C

NMR ($D_2O$) δ: 1.56 (6H, s), 3.24—3.52 (2H, m), 3.36 (3H, s), 4.05—4.60 (3H, m), 4.43 (2H, s), 4.45 (2H, s), 6.88—7.58 (4H, m), 7.30 (2H, s).

## Example 3

(a) 1-[2-(4-Methoxybutyl)phenoxy]-3-[1,1-dimethyl-2-(3-chloro-6-pyridazinyloxy)ethylamino]-2-propanol.

NMR ($CDCl_3$) δ: 1.21 (6H, s), 1.35—2.00 (4H, m), 2.30—3.15 (4H, m), 2.87 (2H, broad s), 3.15—3.56 (2H, m), 3.29 (3H, s), 3.97 (3H, broad s), 4.32 (2H, s), 6.60—7.35 (4H, m), 6.89 (1H, d, J=9Hz), 7.30 (1H, d, J=9Hz).

(b) 1-[2-(4-Methoxybutyl)phenoxy]-3-[1,1-dimethyl-2-(3-isopropylidenehydrazino-6-pyridazinyloxy)ethylamino]-2-propanol.

NMR ($CDCl_3$) δ: 1.28 (6H, s), 1.40—1.85 (4H, m), 1.92 (3H, s), 2.02 (3H, s), 2.35—3.19 (4H, m), 3.20—3.83 (4H, m), 3.29 (3H, s), 3.99 (3H, broad s), 4.29 (2H, s), 6.65—7.40 (4H, m), 6.95 (1H, d, J=9Hz), 7.51 (1H, d, J=9Hz).

## Example 4

(a) 1-(2-Ethoxymethylphenoxy)-3-[1,1-dimethyl-2-(3-chloro-6-pyridazinyloxy)ethylamino]-2-propanol.

NMR ($CDCl_3$) δ: 1.19 (3H, t, J=7Hz), 1.20 (6H, s), 2.50—3.00 (4H, m), 3.50 (2H, q, J=7Hz), 4.00 (3H, broad s), 4.32 (2H, s), 4.48 (2H, s), 6.70—7.43 (4H, m), 6.91 (1H, d, J=9Hz), 7.31 (1H, d, J=9Hz).

(b) 1-(2-Ethoxymethylphenoxy)-3-[1,1-dimethyl-2-(3-hydrazino-6-pyridazinyloxy)ethylamino]-2-propanol hydrochloride.

NMR ($D_2O$) δ: 1.20 (3H, t, J=7Hz), 1.57 (6H, s), 3.12—3.52 (2H, m), 3.60 (2H, q, J=7Hz), 3.99—4.32 (3H, m), 4.43 (2H, s), 4.49 (2H, s), 6.85—7.64 (4H, m), 7.32 (2H, s).

## Example 5

(a) 1-[2-(2-Ethylthioethyl)phenoxy]-3-[1,1-dimethyl-2-(3-chloro-6-pyridazinyloxy)ethylamino]-2-propanol.

NMR ($CDCl_3$) δ: 1.23 (3H, t, J=7Hz), 1.23 (6H, s), 2.55 (2H, q, J=7Hz), 2.56—2.72 (2H, m), 2.67—3.13 (6H, m), 3.98 (3H, s), 4.32 (2H, s), 6.67—7.23 (4H, m), 6.92 (1H, d, J=9Hz), 7.32 (1H, d, J=9Hz).

(b) 1-[2-(2-Ethylthioethyl)phenoxy]-3-[1,1-dimethyl-2-(3-isopropylidenehydrazino-6-pyridazinyloxy)ethylamino]-2-propanol.

NMR ($CD_3OD$) δ: 1.18 (3H, t, J=7Hz), 1.22 (6H, s), 1.92 (3H, s), 1.98 (3H, s), 2.48 (2H, q, J=7Hz),

2.54—3.10 (6H, m), 3.98 (3H, broad s), 4.18 (2H, s), 6.62—7.30 (4H, m), 6.98 (1H, d, J=9Hz), 7.45 (1H, d, J=9Hz).

(c) 1-[2-(2-Ethylthioethyl)phenoxy]-3-[1,1-dimethyl-2-(3-hydrazino-6-pyridazinyloxy)ethylamino]-2-propanol hydrochloride.

Melting point: 181.6—182.5°C

NMR ($CD_3OD$) δ: 1.18 (3H, t, J=7Hz), 1.55 (6H, s), 2.49 (2H, q, J=7Hz), 2.52—3.07 (6H, m), 3.90—4.40 (4H, m), 4.42 (2H, s), 6.66—7.33 (4H, m), 7.43 (2H, s).

Example 6

(a) 1-[2-(2-acetylaminoethyl)phenoxy-3-[1,1-dimethyl-2-(3-chloro-6-pyridazinyloxy)ethylamino]-2-propanol.

NMR ($CDCl_3$) δ: 1.20 (6H, s), 1.93 (3H, s), 2.60—3.18 (4H, m), 2.80 (2H, broad s), 3.18—3.69 (2H, m), 4.00 (3H, broad s), 4.34 (2H, s), 5.98—6.40 (1H, m), 6.69—7.38 (4H, m), 6.97 (1H, d, J=9Hz), 7.35 (1H, d, J=9Hz).

(b) 1-[2-(2-Acetylaminoethyl)phenoxy]-3-[1,1-dimethyl-2-(3-isopropylidenehydrazino-6-pyridazinyloxy)-ethylamino]-2-propanol.

NMR ($CD_3OD$) δ: 1.25 (6H, s), 1.88 (3H, s), 1.95 (3H, s), 2.02 (3H, s), 2.60—3.04 (4H, m), 3.28—3.42 (2H, m), 4.00 (3H, broad s), 4.20 (2H, s), 6.68—7.26 (4H, m), 6.99 (1H, d, J=9Hz), 7.48 (1H, d, J=9Hz).

(c) 1-[2-(2-Acetylaminoethyl)phenoxy]-3-[1,1-dimethyl-2-(3-hydrazino-6-pyridazinyloxy)ethylamino]-2-propanol hydrochloride.

NMR ($CD_3OD$) δ: 1.56 (6H, s), 2.08 (3H, s), 2.80 (2H, t, J=8Hz), 3.18—3.61 (4H, m), 3.96—4.22 (3H, m), 4.44 (2H, s), 6.80—7.30 (4H, m), 7.45 (2H, s).

Example 7

(a) 1-[2-(2-Tetrahydrofurfuryloxyethyl)phenoxy]-3-[1,1-dimethyl-2-(3-chloro-6-pyridazinyloxy)ethylamino]-2-propanol.

NMR ($CDCl_3$) δ: 1.20 (6H, s), 1.38—2.20 (4H, m), 2.66 (2H, broad s), 2.68—3.10 (4H, m), 3.49 (2H, d, J=5Hz), 3.60—4.23 (3H, m), 3.77 (2H, t, J=7Hz), 3.97 (3H, broad s), 4.32 (2H, s), 6.65—7.35 (4H, m), 6.93 (1H, d, J=9Hz), 7.32 (1H, d, J=9Hz).

(b) 1-[2-(2-Tetrahydrofurfuryloxyethyl)phenoxy]-3-[1,1-dimethyl-2-(3-isopropylidenehydrazino-6-pyridazinyloxy)ethylamino]-2-propanol.

NMR ($CDCl_3$) δ: 1.20 (6H, s), 1.45—2.15 (4H, m), 1.91 (3H, s), 2.02 (3H, s), 2.45—3.20 (2H, m), 2.60—3.14 (4H, m), 3.41 (2H, d, J=5Hz), 3.52—4.17 (3H, m), 3.57 (2H, t, J=7Hz), 3.97 (3H, broad s), 4.23 (2H, s, 6.60—7.45 (4H, m), 6.89 (1H, d, J=9Hz), 7.50 (1H, d, J=9Hz).

(c) 1-[2-(2-Tetrahydrofurfuryloxyethyl)phenoxy]-3-[1,1-dimethyl-2-(3-hydrazino-6-pyridazinyloxy)ethylamino]-2-propanol hydrochloride.

Melting point: 168.0—171.0°C

NMR ($D_2O$) δ: 1.40—2.20 (4H, m), 1.56 (6H, s), 2.82 (2H, t, J=7Hz), 3.20—4.01 (9H, m), 4.01—4.40 (3H, m), 4.42 (2H, s), 6.65—7.55 (4H, m), 7.24 (2H, s).

Example 8

(a) 1-[2-(2-Allyloxyethyl)phenoxy]-3-[1,1-dimethyl-2-(3-chloro-6-pyridazinyloxy)ethylamino]-2-propanol.

NMR ($CDCl_3$) δ: 1.21 (6H, s), 2.20—2.75 (2H, m), 2.72—3.17 (4H, m), 3.62 (2H, t, J=6Hz), 3.84—4.25 (2H, m), 3.98 (3H, broad s), 4.33 (2H, s), 4.95—5.40 (2H, m), 5.45—6.40 (1H, m), 6.70—7.37 (4H, m), 6.92 (1H, d, J=9Hz), 7.32 (1H, m, J=9Hz).

(b) 1-[2-(2-Allyloxyethyl)phenoxy]-3-[1,1-dimethyl-2-(3-isopropylidenehydrazino-6-pyridazinyloxy)ethylamino]-2-propanol.

NMR ($CDCl_3$) δ: 1.20 (6H, s), 1.91 (3H, s), 2.02 (3H, s), 2.50—3.15 (2H, m), 2.72—3.10 (4H, m), 3.62 (2H, t, J=6Hz), 3.80—4.18 (2H, m), 3.99 (3H, broad s), 4.23 (2H, s), 4.95—5.41 (2H, m), 5.55—6.30 (1H, m), 6.69—4.40 (4H, m), 6.88 (1H, d, J=9Hz), 7.50 (1H, d, 9Hz).

Example 9

(a) 1-[3-(2-Methoxyethyl)phenoxy]-3-[1,1-dimethyl-2-(3-chloro-6-pyridazinyloxy)ethylamino]-2-propanol.

NMR ($CDCl_3$) δ: 1.21 (6H, s), 2.50—3.12 (4H, m) 2.80 (2H, t, J=7Hz), 3.32 (3H, s), 3.58 (2H, t, J=7Hz), 3.95 (3H, broad s), 4.32 (2H, s), 6.56—6.95 (3H, m), 6.90 (2H, d, J=9Hz), 7.01—7.36 (1H, m), 7.31 (2H, d, J=9Hz).

(b) 1-[3-(2-Methoxyethyl)phenoxy]-3-[1,1-dimethyl-2-(3-hydrazino-6-pyridazinyloxy)ethylamino]-2-propanol hydrochloride.

NMR ($D_2O$) δ: 1.56 (6H, s), 2.83 (3H, t, J=6Hz), 3.19—3.48 (2H, m), 3.33 (3H, s), 3.70 (2H, t, J=6Hz), 4.00—4.28 (3H, m), 4.41 (2H, s), 6.66—7.04 (3H, m), 7.04—7.46 (1H, m), 7.27 (2H, s).

Example 10

(a) 1-[2-Chloro-4-(2-methoxyethyl)phenoxy]-3-[1,1-dimethyl-2-(3-chloro-6-pyridazinyloxy)ethylamino]-2-propanol.

NMR ($CDCl_3$) δ: 1.21 (6H, s), 2.54—3.02 (6H, m), 3.32 (3H, s), 3.54 (2H, t, J=6Hz), 3.98 (3H, broad s), 4.32 (2H, s), 6.65—7.28 (3H, m), 6.90 (1H, d, J=9Hz), 7.29 (1H, d, J=9Hz).

(b) 1-[2-Chloro-4-(2-methoxyethyl)phenoxy]-3-[1,1-dimethyl-2-(3-hydrazino-6-pyridazinyloxy)ethylamino]-2-propanol hydrochloride.

NMR ($D_2O$) δ: 1.57 (6H, s), 2.83 (2H, t, J=6Hz), 3.36.

Example 11

(a) To a suspension of 1.32 g of 61% sodium hydride in 20 ml of benzene was added dropwise at room temperature a solution of 2.6 g of 2-methyl-2-aminopropanethiol in 10 ml of benzene. Then, a solution of 4.1 g of 3,6-dichloropyridazine in 10 ml of benzene was added, and the mixture was refluxed for 6 hours. After cooling, the reaction mixture was washed with water and dried over magnesium sulfate. The solvent was evaporated to give 1.96 g of 3-(2-amino-2-methylpropylthio)-6-chloropyridazine.

NMR ($CDCl_3$) δ: 1.24 (6H, s), 1.42 (2H, s), 3.51 (2H, s), 7.15 (1H, d, J=9Hz), 7.35 (1H, d, J=9Hz).

(b) A solution of 980 mg of 3-(2-amino-2-methylpropylthio)-6-chloropyridazine and 960 mg of 1-[2-(2-methoxyethyl)phenoxy]-2,3-epoxypropane in 60 ml of methanol was treated in the same way as in Example 1, (b) to give 534 mg of 1-[2-(2-methoxyethyl)phenoxy]-3-[1,1-dimethyl-2-(3-chloro-6-pyridazinylthio)ethylamino]-2-propanol.

NMR ($CDCl_3$) δ: 1.25 (6H, s), 2.34—2.75 (2H, m), 2.70—3.10 (4H, m), 3.32 (3H, s), 3.58 (2H, s), 3.58 (2H, t, J=6Hz), 3.98 (3H, broad s), 6.69—7.40 (4H, m), 7.21 (2H, s).

(c) A mixture of 525 mg of 1-[2-(2-methoxyethyl)phenoxy]-3-[1,1-dimethyl-2-(3-chloro-6-pyridazinyloxy)ethylamino]-2-propanol obtained in (b) above, 12 ml of hydrazine hydrate and 12 ml of ethanol was treated in the same way as in Example 1, (c) to give 498 mg of 1-[2-(2-methoxyethyl)phenoxy]-3-[1,1-dimethyl-2-(3-isopropylidenehydrazino-6-pyridazinylthio)ethylamino]-2-propanol.

NMR ($CDCl_3$) δ: 1.21 (6H, s), 1.89 (3H, s), 2.01 (3H, s), 2.10—3.10 (2H, m), 2.56—3.10 (4H, m), 3.30 (3H, s), 3.46 (2H, s), 3.57 (2H, t, J=6Hz), 3.96 (3H, broad s), 6.65—7.35 (6H, m).

(d) A mixture of 575 mg of 1-[2-(2-methoxyethyl)phenoxy]-3-[1,1-dimethyl-2-(3-isopropylidenehydrazino-6-pyridazinylthio)ethylamino]-2-propanol obtained in (c) above, 12 ml of hydrazine hydrate and 12 ml of ethanol was treated in the same way as in Example 1, (d) to give 550 mg of 1-[2-(2-methoxyethyl)phenoxy]-3-[1,1-dimethyl-2-(3-hydrazino-6-pyridazinylthio)ethylamino]-2-propanol hydrochloride.

NMR ($D_2O$) δ: 1.59 (6H, s), 2.90 (2H, t, J=7Hz), 3.25—3.90 (4H, m), 3.38 (3H, s), 3.71 (2H, s), 4.02—4.48 (3H, m), 6.82—7.53 (4H, m), 7.25 (1H, d, J=9Hz), 7.55 (1H, d, J=9Hz).

In the same way as in Example 11, the compounds shown in Examples 12 to 14 were obtained.

Example 12

(a) 1-(2-Methylphenoxy)-3-[1,1-dimethyl-2-(3-chloro-6-pyridazinylthio)ethylamino]-2-propanol.

NMR ($CDCl_3$) δ: 1.23 (6H, s), 2.19 (3H, s), 2.56 (2H, broad s), 2.72—2.90 (2H, m), 3.56 (2H, s), 3.97 (3H, broad s), 6.60—7.40 (4H, m), 7.18 (2H, s).

(b) 1-(2-Methylphenoxy)-3-[1,1-dimethyl-2-(3-hydrazino-6-pyridazinylthio)ethylamino]-2-propanol hydrochloride.

Melting point: 207—210°C

NMR ($D_2O$) δ: 1.57 (6H, s), 2.20 (3H, s), 3.30—3.54 (2H, m), 3.68 (2H, s), 4.04—4.50 (3H, m), 6.80—7.35 (4H, m), 7.14 (1H, d, J=9Hz), 7.45 (1H, d, J=9Hz).

Example 13

(a) 1-(2-Chloro-3-methylphenoxy)-3-[1,1-dimethyl-2-(3-chloro-6-pyridazinylthio)ethylamino]-2-propanol.

NMR ($CDCl_3$) δ: 1.25 (6H, s), 2.37 (3H, s), 2.50—2.80 (2H, m), 2.77—2.95 (2H, m), 3.57 (2H, s), 4.00 (3H, broad s), 6.62—7.15 (3H, m), 7.19 (2H, s).

(b) 1-(2-Chloro-3-methylphenoxy)-3-[1,1-dimethyl-2-(3-hydrazino-6-pyridazinylthio)ethylamino]-2-propanol hydrochloride.

NMR ($D_2O$) δ: 1.67 (6H, s), 2.40 (3H, s), 3.43—3.70 (2H, m), 3.75 (2H, s), 4.10—4.65 (3H, m), 6.80—7.48 (3H, m), 7.19 (1H, d, J=9Hz), 7.45 (1H, d, J=9Hz).

Example 14

(a) 1-(2-Chloro-5-methylphenoxy)-3-[1,1-dimethyl-2-(3-chloro-6-pyridazinylthio)ethylamino]-2-propanol.

NMR ($CDCl_3$) δ: 1.22 (6H, s), 2.30 (3H, s), 2.40—2.79 (2H, m), 2.78—2.95 (2H, m), 3.56 (2H, s), 3.98 (3H, broad s), 6.66 (1H, d, J=8Hz), 6.72 (1H, s), 7.15 (1H, d, J=8Hz), 7.18 (2H, s).

(b) 1-(2-Chloro-5-methylphenoxy)-3-[1,1-dimethyl-2-(3-isopropylidenehydrazino-6-pyridazinylthio)ethylamino]-2-propanol.

NMR ($CDCl_3$) δ: 1.22 (6H, s), 1.92 (3H, s), 2.03 (3H, s), 2.30 (3H, s), 2.72—2.90 (2H, m), 2.45—3.15 (2H, m), 3.48 (2H, s), 4.00 (3H, broad s), 6.68 (1H, d, J=8Hz), 6.73 (1H, s), 7.17 (1H, d, J=8Hz), 7.25 (2H, s).

(c) 1-(2-Chloro-5-methylphenoxy)-3-[1,1-dimethyl-2-(3-hydrazino-6-pyridazinylthio)ethylamino]-2-propanol hydrochloride.

NMR ($D_2O$) δ: 1.61 (6H, s), 2.36 (3H, s), 3.51 (2H, d, J=5Hz), 3.72 (2H, s), 4.08—4.57 (3H, m), 6.70—7.60 (3H, m), 7.15 (1H, d, J=9Hz), 7.41 (1H, d, J=9Hz).

Example 15

(a) A mixture of 4.3 g of 1-(2-methylphenoxy)-2,3-epoxypropane, 7.03 g of N,N-dibenzyl-2-methyl-1,2-propane-diamine and 50 ml of ethanol was refluxed for 20 hours, and the solvent was evaporated under reduced pressure. To a benzene solution of the residue was added 1N hydrochloric acid, to deposit an oily material. After removal of the benzene layer, the residue was extracted with chloroform. The organic layer was washed with 5% sodium carbonate and dried over anhydrous sodium sulfate. The solvent was evaporated to give a colorless oily product. The product was chromatographed on a column of silica gel using chloroform as an eluent. 7.3 g of 1-(2-methylphenoxy)-3-(1,1-dimethyl-2-dibenzylaminoethylamino)-2-propanol was obtained from the eluate.

NMR ($CDCl_3$) δ: 1.00 (6H, s), 2.20 (3H, s), 2.40—2.80 (4H, m), 2.54 (2H, s), 3.62 (4H, s), 3.80 (3H, broad s), 6.60—7.42 (4H, m), 7.26 (10H, s).

(b) Concentrated hydrochloric acid (1.4 ml) and 5% palladium carbon was added to a solution of 3 g of 1-(2-methylphenoxy)-3-(1,1-dimethyl-2-dibenzylaminoethylamino)-2-propanol in 10 ml of ethanol, and the mixture was hydrogenated at room temperature for 24 hours. After the catalyst was removed by filtration, the filtrate was concentrated under reduced pressure. The residue was dissolved in water, made alkaline with potassium carbonate, then extracted with chloroform, and dried over anhydrous magnesium sulfate. 1.585 g of 1-(2-methylphenoxy)-3-(1,1-dimethyl-2-aminoethylamino)-2-propanol was obtained as a colorless oil.

NMR ($CDCl_3$) δ: 1.03 (6H, s), 2.08 (4H, broad s), 2.22 (3H, s), 2.56 (2H, s), 2.63—2.83 (2H, m), 3.97 (3H, broad s), 6.65—7.30 (4H, m).

(c) To 1.58 g of 1-(2-methylphenoxy)-3-(1,1-dimethyl-2-aminoethylamino)-2-propanol heated at 110°C in an oil bath with stirring was added 934 mg of 3,6-dichloropyridazine little by little. After heating at 110°C for 1 hour, the reaction mixture was dissolved in benzene, and extracted with 1N hydrochloric acid. The aqueous layer was made alkaline with potassium carbonate, and extracted with chloroform. The chloroform layer was dried over anhydrous magnesium sulfate, and the solvent was evaporated to give 1.55 g of 1-(2-methylphenoxy)-3-[1,1-dimethyl-2-(3-chloro-6-pyridazinylamino)ethylamino]-2-propanol as an oil.

NMR ($CDCl_3$) δ: 1.24 (6H, s), 2.13 (3H, s), 2.77—3.03 (2H, m), 3.30—3.58 (2H, d, J=6Hz), 3.69—4.32 (6H, m), 6.62—7.32 (4H, m), 6.73 (1H, d, J=9Hz), 7.00 (1H, d, J=9Hz).

(d) To a solution of 140 mg of 1-(2-methylphenoxy)-3-[1,1-dimethyl-2-(3-chloro-6-pyradazinylamino)ethylamino]-2-propanol in ethanol was added a saturated ether solution of hydrogen chloride. After removal of the solvent, 80 mg of ethyl carbazinate was added to the residue. The mixture was heated at 140°C for 2 hours, then dissolved in chloroform, washed with 5% sodium carbonate, and dried over anhydrous sodium sulfate. The resulting crude product was purified by thin-layer chromatography [silica gel (Merck $GF_{254}$); chloroform/methanol = 4/1] to give 66 mg of 1-(2-methylphenoxy)-3-[1,1-dimethyl-2-(3-ethoxycarbonylhydrazino-6-pyridazinylamino)ethylamino]-2-propanol.

(e) A solution of 66 mg of 1-(2-methylphenoxy)-3-[1,1-dimethyl-2-(3-ethoxycarbonylhydrazino-6-pyridazinylamino)-ethylamino]-2-propanol in 5 ml of 10% hydrochloric acid was refluxed for 5 hours. After the solvent was evaporated under reduced pressure, the residue was recrystallized from ethanol to give 31 mg of 1-(2-methylphenoxy)-3-[1,1-dimethyl-2-(3-hydrazino-6-pyridazinylamino)ethylamino]-2-propanol hydrochloride as colorless crystals.

Melting point: 164.4—166.9°C.

NMR ($D_2O$) δ: 1.53 (6H, s), 2.21 (3H, s), 3.48 (2H, d, J=5Hz), 3.75 (2H, broad s), 4.13 (2H, d, J=5Hz), 4.20—4.60 (1H, m), 6.70—7.35 (4H, m), 7.09 (1H, d, J=9Hz), 7.29 (1H, d, J=9Hz).

(f) A solution of 1-(2-methylphenoxy)-3-[1,1-dimethyl-2-(3-hydrazino-6-pyridazinylamino)ethylamino]-2-propanol hydrochloride in water was made alkaline with potassium carbonate, extracted with chloroform and dried over anhydrous sodium sulfate. The resulting free base was treated with acetone to give 1-(2-methylphenoxy)-3-[1,1-dimethyl-2-(3-isopropylidenehydrazino-6-pyridazinylamino)ethylamino]-2-propanol.

NMR ($CDCl_3$) δ: 1.17 (6H, s), 1.87 (3H, s), 1.98 (3H, s), 2.16 (3H, s), 2.70—3.05 (2H, m), 3.33 (2H, broad s), 4.01 (3H, broad s), 6.50—7.30 (4H, m), 6.62 (1H, d, J=9Hz), 7.32 (1H, d, J=9Hz).

Example 16

In the same way as in Example 15, the following compounds were obtained.

(a) 1-(2-Chlorophenoxy)-3-(1,1-dimethyl-2-dibenzylamino-ethylamino)-2-propanol.

NMR ($CDCl_3$) δ: 1.00 (6H, s), 2.36 (2H, broad s), 2.43—2.65 (2H, m), 2.52 (2H, s), 3.63 (4H, s), 3.87 (3H, broad s), 6.68—7.46 (4H, m), 7.28 (10H, s).

(b) 1-(2-Chlorophenoxy)-3-(1,1-dimethyl-2-aminoethylamino)-2-propanol.

NMR ($CDCl_3$) δ: 1.05 (6H, s), 2.17 (4H, broad s), 2.57 (2H, s), 2.60—2.95 (2H, m), 3.97 (3H, broad s), 6.70—7.45 (4H, m).

(c) 1-(2-Chlorophenoxy)-3-[1,1-dimethyl-2-(3-hydrazino-6-pyridazinylamino)ethylamino]-2-propanol hydrochloride

Melting point: 163.4—166.9°C

NMR ($D_2O$) δ: 1.52 (6H, s), 3.44 (2H, d, J=5Hz), 3.74 (2H, s), 4.14 (2H, d, J=5Hz), 4.20—4.50 (1H, m), 6.86—7.60 (4H, m), 7.20 (1H, d, J=9Hz), 7.39 (1H, d, J=9Hz).

Example 17

(a) In the same way as in Example 15, (a), 1-(2-methylphenoxy)-3-[1,1-dimethyl-2-(N-methylbenzylamino)-ethylamino]-2-propanol was produced by using N-benzyl-N-methyl-2-methyl-1,2-propanediamine instead of N,N-dibenzyl-2-methyl-1,2-propanediamine.

NMR ($CDCl_3$) δ: 1.08 (6H, s), 2.21 (3H, s), 2.26 (3H, s), 2.43 (2H, s), 2.62—2.88 (2H, m), 2.91 (2H, broad s), 3.60 (2H, s), 3.93 (3H, broad s), 6.65—7.40 (4H, m), 7.23 (5H, s).

The following compounds (b), (c), (d), (e) and (f) were produced in the same way as in Example 15, (b), (c), (d), (e) and (f) respectively by using 1-(2-methylphenoxy-3-[1,1-dimethyl-2-(N-methylbenzylamino)-ethylamino]-2-propanol obtained in (a) above.

(b) 1-(2-Methylphenoxy)-3-(1,1-dimethyl-2-methylaminoethylamino)-2-propanol.

NMR ($CDCl_3$) δ: 1.08 (6H, s), 2.23 (3H, s), 2.44 (3H, s), 2.48 (5H, s), 2.62—2.88 (2H, m), 3.96 (3H, broad s), 6.65—7.30 (4H, m).

(c) 1-(2-Methylphenoxy)-3-[1,1-dimethyl-2-[N-(3-chloro-6-pyridazinyl)methylamino]ethylamino]-2-propanol.

NMR ($CDCl_3$) δ: 1.18 (6H, s), 2.20 (3H, s), 2.67 (2H, broad s), 2.78—3.00 (2H, m), 3.13 (3H, s), 3.68 (2H, s), 3.96 (3H, broad s), 6.60—7.30 (4H, m), 6.80 (1H, d, J=9Hz), 7.12 (1H, d, J=9Hz).

(d) 1-(2-Methylphenoxy)-3-[1,1-dimethyl-2-[N-(3-ethoxycarbonylhydrazino-6-pyridazinyl)methylamino]-ethylamino]-2-propanol.

NMR ($CDCl_3$) δ: 1.16 (6H, s), 1.22 (3H, t, J=7Hz), 2.20 (3H, s), 2.50—3.30 (4H, m), 3.10 (3H, s), 3.55 (2H, s), 3.96 (3H, broad s), 4.14 (2H, q, J=7Hz), 6.60—7.30 (4H, m), 6.85 (2H, s).

(e) 1-(2-Methylphenoxy)-3-[1,1-dimethyl-2-[N-(3-hydrazino-6-pyridazinyl)methylamino]ethylamino]-2-propanol.

NMR ($D_2O$) δ: 1.52 (5H, s), 2.20 (3H, s), 3.29 (3H, s), 3.15—3.58 (2H, m), 3.93 (2H, s), 4.00—4.50 (3H, m), 6.80—7.35 (4H, m), 7.29 (1H, d, J=9Hz), 7.68 (1H, d, J=9Hz).

(f) 1-(2-Methylphenoxy)-3-[1,1-dimethyl-2-[N-(3-isopropylidenehydrazino-6-pyridazinyl)methylamino]-ethylamino]-2-propanol.

NMR ($CDCl_3$) δ: 1.16 (6H, s), 1.88 (3H, s), 2.00 (3H, s), 2.20 (3H, s), 2.66—3.40 (4H, m), 3.10 (3H, s), 3.57 (2H, s), 3.97 (3H, broad s), 6.60—7.40 (4H, m), 6.75 (1H, d, J=9Hz), 7.36 (1H, d, J=9Hz).

Example 18

In the same way as in Example 15, the following compounds (a), (b), (c), (d) and (e) were obtained.

(a) 1-(2-Methylphenoxy)-3-[1-methyl-3-(N-methylbenzylamino)-propylamino]-2-propanol.

NMR ($CDCl_3$) δ: 1.05 (3H, d, J=6Hz), 1.58 (2H, q, J=7Hz), 2.18 (3H, s), 2.22 (3H, s), 2.46 (2H, t, J=7Hz), 2.50—2.96 (5H, m), 3.45 (2H, m), 3.97 (3H, broad s), 6.65—7.40 (4H, m), 7.27 (5H, s).

(b) 1-(2-Methylphenoxy)-3-(1-methyl-3-methylaminopropylamino)-2-propanol.

NMR ($CDCl_3$) δ: 1.07 (3H, d, J=7Hz), 1.55 (2H, q, J=6Hz), 2.21 (3H, s), 2.41 (6H, s), 2.68 (2H, t, J=7Hz), 2.10—3.00 (3H, m), 3.98 (3H, broad s), 6.60—7.30 (4H, m).

(c) 1-(2-Methylphenoxy)-3-[1-methyl-3-[N-(3-chloro-6-pyridazinyl)methylamino]propylamino]-2-propanol.

NMR ($CDCl_3$) δ: 1.14 (3H, d, J=6Hz), 1.22 (2H, q, J=7Hz), 2.21 (3H, s), 2.40—3.00 (3H, m), 2.70 (2H, broad

s), 3.08 (3H, s), 3.50—3.90 (2H, m), 4.02 (3H, broad s), 6.62—7.40 (4H, m), 6.74 (1H, d, J=9.5Hz), 7.14 (1H, d, J=9.5Hz).

(d) 1-(2-Methylphenoxy)-3-[1-methyl-3-[N-(3-ethoxycarbonylhydrazino-6-pyridazinyl)methylamino]propylamino]-2-propanol.

NMR ($CDCl_3$) δ: 1.11 (3H, d, J=6Hz), 1.21 (3H, t, J=7Hz), 1.66 (2H, q, J=7Hz, 2.20 (3H, s), 2.50—3.10 (3H, m), 2.99 (3H, s), 3.30—3.82 (2H, m), 4.00 (3H, broad s), 4.14 (2H, q, J=7Hz), 6.60—7.30 (4H, m), 6.81 (2H, s).

(e) 1-(2-Methylphenoxy)-3-[1-methyl-3-[N-(3-hydrazino-6-pyridazinyl)methylamino]propylamino]-2-propanol hydrochloride.

NMR ($D_2O$) δ: 1.47 (3H, d, J=6Hz), 1.80—2.40 (2H, m), 2.22 (3H, s), 2.70—3.90 (5H, m), 3.23 (3H, s), 4.00—4.60 (3H, m), 6.70—7.90 (6H, m).

Example 19

In the same way as in Example 15, the following compounds (a), (b), (c), (d) and (e) were obtained.

(a) 1-(2-Methylphenoxy)-3-(1-methyl-3-dibenzylaminopropylamino)-2-propanol.

NMR ($CDCl_3$) δ: 0.94 (3H, d, 7=6Hz), 1.33—1.85 (2H, m), 2.21 (3H, s), 2.30—2.92 (5H, m), 2.48 (2H, t, J=7Hz), 3.52 (4H, s), 3.90 (3H, broad s), 6.65—7.50 (4H, m), 7.28 (10H, s).

(b) 1-(2-Methylphenoxy)-3-(1-methyl-3-aminopropylamino)-2-propanol.

NMR ($CDCl_3$) δ: 1.09 (3H, d, J=6Hz), 1.54 (2H, q, J=6Hz), 2.21 (3H, s), 2.30—3.03 (5H, m), 3.30 (4H, s), 3.98 (3H, broad s), 6.62—7.38 (4H, m).

(c) 1-(2-Methylphenoxy)-3-[1-methyl-3-(3-chloro-6-pyridazinylamino)propylamino]-2-propanol.

NMR ($CDCl_3$) δ: 1.15 (3H, d, J=6Hz), 1.74 (2H, q, J=6Hz), 2.20 (3H, s), 2.60—3.30 (6H, m), 3.49 (2H, t, J=6Hz), 4.01 (3H, broad s), 6.59 (1H, d, J=9Hz), 6.63—7.38 (4H, m), 7.05 (1H, d, J=9Hz).

(d) 1-(2-Methylphenoxy)-3-[1-methyl-3-(3-ethoxycarbonylhydrazino-6-pyridazinylamino)propylamino]-2-propanol.

Melting point: 115.4—118.8°C

NMR ($CDCl_3$) δ: 1.13 (3H, d, J=6Hz), 1.24 (3H, t, J=7Hz), 1.68 (2H, q, J=7Hz), 2.23 (3H, s), 2.62—3.07 (3H, m), 3.38 (2H, t, J=7Hz), 3.70—6.00 (5H, m), 4.02 (3H, broad s), 4.16 (2H, q, J=7Hz), 6.55 (1H, d, J=9.5 Hz), 6.58—7.40 (4H, m), 6.95 (1H, d, J=9.5Hz).

(e) 1-(2-Methylphenoxy)-3-[1-methyl-3-(3-hydrazino-6-pyridazinylamino)propylamino]-2-propanol hydrochloride.

Melting point: 169.0—171.6°C

NMR ($D_2O$) δ: 1.45 (3H, d, J=6Hz), 1.98—2.45 (2H, m), 2.23 (3H, s), 3.17—3.85 (5H, m), 4.00—4.60 (3H, m), 6.73—7.40 (4H, m), 7.47 (2H, s).

Some examples for the preparation of drugs containing the compounds of this invention are shown below.

Example A

Tablets:—

Tablets containing 5 mg or 20 mg, per tablet, of the active compound of this invention were prepared in accordance with the following recipes.

Recipe 1-a (5 mg tablets)

| Ingredients | mg/tablet |
|---|---|
| 1-[2-(2-methoxyethyl)phenoxy]-3-[1,1-dimethyl-2-(3-hydrazino-6-pyridazinyloxy)ethylamino]-2-propanol hydrochloride | 5 |
| Lactose | 137.2 |
| Starch | 44.8 |
| Carboxymethylcellulose calcium | 10 |
| Talc | 2 |
| Magnesium stearate | 1 |
| | 200.0 mg |

Recipe 1-b (20 mg tablets)

| Ingredients | mg/tablet |
| --- | --- |
| 1-[2-(2-methoxyethyl)phenoxy]-3-[1,1-dimethyl-2-(3-hydrazino-6-pyridazinyloxy)ethylamino]-2-propanol hydrochloride | 20 |
| Lactose | 122.2 |
| Starch | 44.8 |
| Carboxymethylcellulose calcium | 10 |
| Talc | 2 |
| Magnesium stearate | 1 |
| | 200.0 mg |

Specifically, crystals of the 1-[2-(2-methoxyethyl)phenoxy]-3-[1,1-dimethyl-2-(3-hydrazino-6-pyridazinyloxy)ethylamino]-2-propanol hydrochloride were pulverized, and well mixed with lactose and starch. A 10% starch paste was added to the mixture and they were mixed with stirring to prepare granules. After drying, the granules were adjusted to a particle diameter of about 840 microns and mixed with talc and magnesium stearate. The mixture was tableted.

Example B

Capsules:—

Recipe 2 (20 mg capsule)

| Ingredients | mg/tablet |
| --- | --- |
| 1-[2-(2-methoxyethyl)phenoxy]-3-[1,1-dimethyl-2-(3-hydrazino-6-pyridazinyloxy)ethylamino]-2-propanol hydrochloride | 20 |
| Lactose | 57.8 |
| Starch | 30 |
| Magnesium stearate | 2.2 |
| | 110.0 mg |

Crystals of the 1-[2-(2-methoxyethyl)phenoxy]-3-[1,1-dimethyl-2-(3-hydrazino-6-pyridazinyloxy)ethylamino]-2-propanol hydrochloride were well pulverized and mixed with starch, lactose and magnesium stearate. After good mixing, the mixture was filled in No. 5 capsules.

**Claims for the Contracting States: BE CH DE FR GB IT LI LU NL SE**

1. A hydrazinopyridazine compound represented by the formula

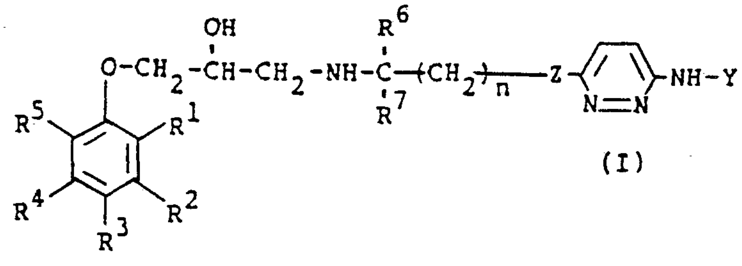

(I)

wherein

$R^6$ and $R^7$, independently from each other, represent a hydrogen atom or a methyl group,

Z represents —O—, —S—, or

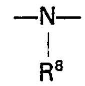

in which $R^8$ represents a hydrogen atom or a $C_1$—$C_5$ alkyl group;

Y represents $—NH_2$, $—NH—COOC_2H_5$, or

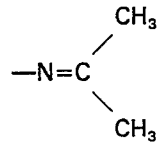 ;

n is 1 or 2; and

(a) when Z represents —O—,

$R^1$ represents a hydrogen atom, a $C_1$—$C_5$ alkyl group substituted by a $C_1$—$C_5$ alkoxy, $C_1$—$C_5$ alkylthio, $C_2$—$C_5$ alkanoylamino, allyloxy or tetrahydrofurfuryloxy group, a $C_1$—$C_5$ alkoxy group substituted by a 2-furyl, phenyl or $C_1$—$C_5$ alkoxy group,

$R^2$ and $R^3$, independently from each other, represent a hydrogen atom or a $C_1$—$C_5$ alkyl group substituted by a $C_1$—$C_5$ alkoxy group,

with proviso that $R^1$, $R^2$ and $R^3$ are not simultaneously hydrogen atoms, and that when $R^3$ represents a $C_1$—$C_5$ alkyl group substituted by a $C_1$—$C_5$ alkoxy group, $R^1$ may represent a halogen atom, and

$R^4$ and $R^5$ represent a hydrogen atom; and

(b) when Z represents —S— or

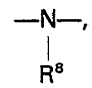,

$R^1$ represents a methoxyethyl or $C_1$—$C_5$ alkyl group, or a halogen atom,

$R^2$ and $R^4$, independently from each other, represent a halogen atom or a methyl group, and

$R^3$ and $R^5$ represent a hydrogen atom, or its salt.

2. The compound of claim 1 wherein both $R^6$ and $R^7$ are methyl groups.

3. The compound. of claim 1 or 2 as pharmaceutically active substances.

4. The compound of claim 1 or 2 as an antihypertensive agent.

5. A process for producing the compound of the formula (I) given in claim 1, which comprises reacting a compound represented by the formula

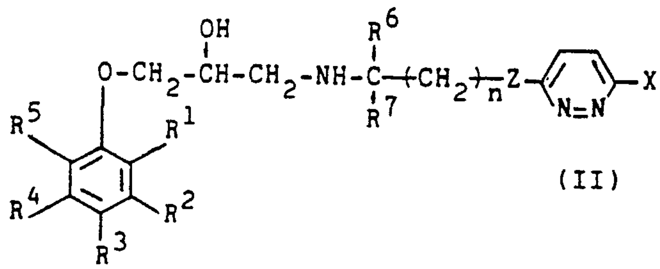

(II)

wherein X represents a halogen atom, and $R^1$, $R^2$, $R^3$, $R^4$, $R^5$, $R^6$, $R^7$, Z and n are as defined in claim 1, with (a) hydrazine or hydrazine hydrate when Z in formula (II) represents —O— or —S—, or (b) ethyl carbazinate when Z in formula (II) represents

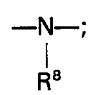;

then as necesssary, hydrolyzing the resulting compound when it corresponds to formula (I) in which Y is $—NH—COOC_2H_5$; and as necessary reacting the resulting compound with acetone when it corresponds to formula (I) in which Y is $—NH_2$; and as necessary further, converting the compound of formula (I) to its salt.

6. A medicament containing a compound of the formula (I) given in claim 1 or a pharmaceutically acceptable salt thereof and a pharmaceutically acceptable carrier or diluent.

**Claim for the Contracting State: AT**

A process for producing a hydrazinopyridazine compound represented by the formula

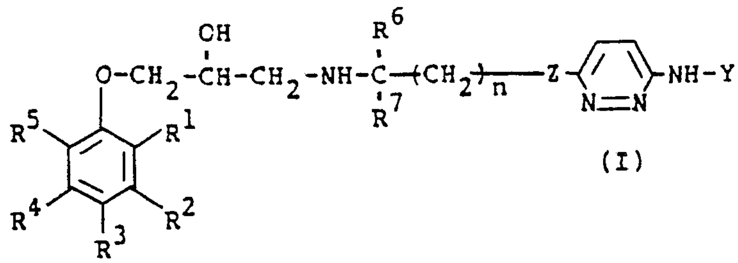

(I)

wherein

$R^6$ and $R^7$, independently from each other, represent a hydrogen atom or a methyl group,

Z represents —O—, —S—, or

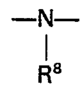

in which $R^8$ represents a hydrogen atom or a $C_1$—$C_5$ alkyl group;

Y represents $—NH_2$, $—NH—COOC_2H_5$, or

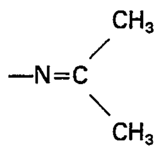 ;

n is 1 or 2; and

(a) when Z represents —O—,

$R^1$ represents a hydrogen atom, a $C_1$—$C_5$ alkyl group substituted by a $C_1$—$C_5$ alkoxy, $C_1$—$C_5$ alkylthio, $C_2$—$C_5$ alkanoylamino, allyloxy or tetrahydrofurfuryloxy group, a $C_1$—$C_5$ alkoxy group substituted by a 2-furyl, phenyl or $C_1$—$C_5$ alkoxy group,

$R^2$ and $R^3$, independently from each other, represent a hydrogen atom or a $C_1$—$C_5$ alkyl group substituted by a $C_1$—$C_5$ alkoxy group,

with proviso that $R^1$, $R^2$ and $R^3$ are not simultaneously hydrogen atoms, and that when $R^3$ represents a $C_1$—$C_5$ alkyl group substituted by a $C_1$—$C_5$ alkoxy group, $R^1$ may represent a halogen atom, and

$R^4$ and $R^5$ represent a hydrogen atom; and

(b) when Z represents —S— or

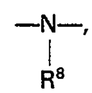

$R^1$ represents a methoxyethyl or $C_1$—$C_5$ alkyl group, or a halogen atom,

$R^2$ and $R^4$, independently from each other, represent a halogen atom or a methyl group,

$R^3$ and $R^5$ represent a hydrogen atom, or its salt, which comprises reacting a compound represented by the formula

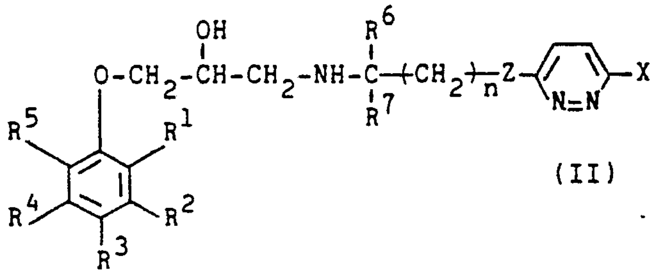

(II)

wherein X represents a halogen atom, and $R^1$, $R^2$, $R^3$, $R^4$, $R^5$, $R^6$, $R^7$, Z and n are as defined above, with (a)

hydrazine or hydrazine hydrate when Z in formula (II) represents —O— or —S—, or (b) ethyl carbazinate when Z in formula (II) represents

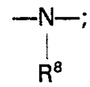

then as necesssary, hydrolyzing the resulting compound when it corresponds to formula (I) in which Y is —NH—$COOC_2H_5$; and as necessary reacting the resulting compound with acetone when it corresponds to formula (I) in which Y is —$NH_2$; and as necessary further, converting the compound of formula (I) to its salt.

**Patentansprüche für die Vertragsstaaten: BE CH DE FR GB IT LI LU NL SE**

1. Hydrazinopyridazinverbindung der Formel

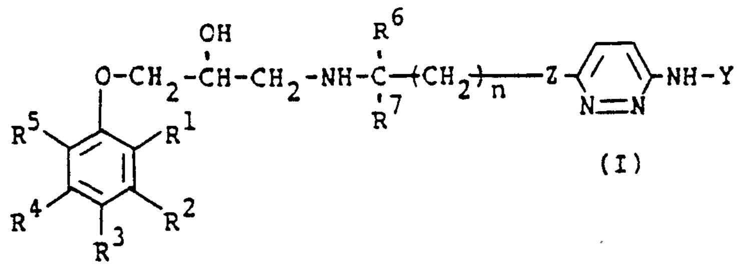

(I)

worin
$R^6$ und $R^7$ unabhängig voneinander für ein Wasserstoffatom oder eine Methylgruppe stehen,
Z für —O—, —S— oder

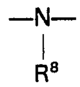

steht, wobei $R^8$ für ein Wasserstoffatom oder eine $C_1$—$C_5$-Alkylgruppe steht,
Y für —$NH_2$, —NH—$COOC_2H_5$ oder

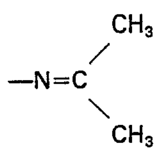

steht,
n den Wert 1 oder 2 hat, und
(a) wenn Z für —O— steht,
$R^1$ für ein Wasserstoffatom, eine $C_1$—$C_5$-Alkylgruppe, substituiert durch eine $C_1$—$C_5$-Alkoxy-, $C_1$—$C_5$-Alkylthio-, $C_2$—$C_5$-Alkanoylamino-, Allyloxy- oder Tetrahydrofurfuryloxygruppe, eine $C_1$—$C_5$-Alkoxygruppe, substituiert durch eine 2-Furyl-, Phenyl- oder $C_1$—$C_5$-Alkoxygruppe, steht,
$R^2$ und $R^3$ unabhängig voneinander für ein Wasserstoffatom oder eine $C_1$—$C_5$-Alkylgruppe, substituiert durch eine $C_1$—$C_5$-Alkoxygruppe, stehen,
mit der Maßgabe, daß $R^1$, $R^2$ und $R^3$ nicht gleichzeitig Wasserstoffatome sind und daß, wenn $R^3$ für eine $C_1$—$C_5$-Alkylgruppe, die durch eine $C_1$—$C_5$-Alkoxygruppe substituiert ist, steht, $R^1$ ein Halogenatom sein kann, und
$R^4$ und $R^5$ ein Wasserstoffatom darstellen und
(b) wenn Z für —S— oder

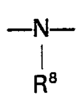

steht,
$R^1$ für eine Methoxyethyl- oder $C_1$—$C_5$-Alkylgruppe oder ein Halogenatom steht,
$R^2$ und $R^4$ unabhängig voneinander für ein Halogenatom oder eine Methylgruppe stehen, und
$R^3$ und $R^5$ ein Wasserstoffatom darstellen,
oder ihr Salz.

2. Verbindung nach Anspruch 1, dadurch gekennzeichnet, daß beide Gruppen $R^6$ und $R^7$ Methylgruppen sind.

3. Verbindung nach Anspruch 1 oder 2 als pharmazeutische Wirkstoffe.

4. Verbindung nach Anspruch 1 oder 2 als Antihypertonikum.

5. Verfahren zur Herstellung der Verbindung der Formel (I) nach Anspruch 1, dadurch gekennzeichnet, daß man eine Verbindung der Formel

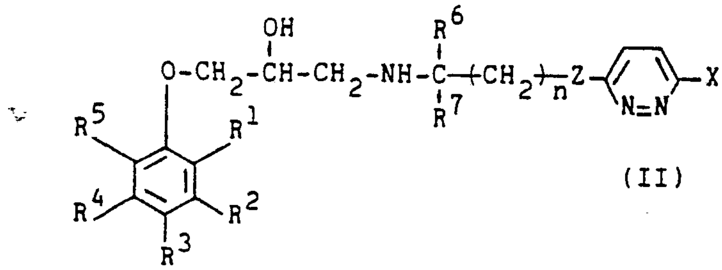

(II)

worin X für ein Halogenatom steht und $R^1$, $R^2$, $R^3$, $R^4$, $R^5$, $R^6$, $R^7$, Z und n wie in Anspruch 1 definiert sind, mit (a) Hydrazin oder Hydrazinhydrat, wenn Z in Formel (II) für —O— oder —S— steht, oder (b) Ethylcarbazinat, wenn Z in Formel (II) für

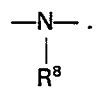

steht umsetzt, sodann erforderlichenfalls die resultierende Verbindung, wenn sie Formel (I), worin Y für —NH—$COOC_2H_5$ steht, entspricht, hydrolysiert und erforderlichenfalls die resultierende Verbindung, wenn sie der Formel (I), bei der Y für —$NH_2$ steht, entspricht, mit Aceton umsetzt und weiterhin erforderlichenfalls die Verbindung der Formel (I) in ihr Salz umwandelt.

6. Arzneimittel, dadurch gekennzeichnet, daß es eine Verbindung der Formel (I) nach Anspruch 1 oder ein pharmazeutisch annehmbares Salz davon und einen pharmazeutisch annehmbaren Träger oder Verdünnungsmittel enthält.

**Patentanspruch für den Vertragsstaat: AT**

Verfahren zur Herstellung einer Hydrazinopyridazinverbindung der Formel

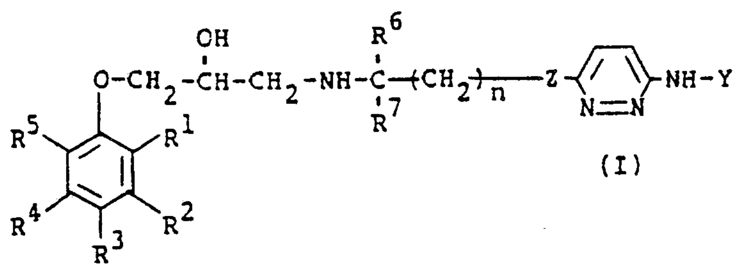

(I)

worin

$R^6$ und $R^7$ unabhängig voneinander für ein Wasserstoffatom oder eine Methylgruppe stehen,

Z für —O—, —S— oder

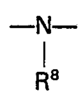

steht, wobei $R^8$ für ein Wasserstoffatom oder eine $C_1$—$C_5$-Alkylgruppe steht,

Y für —$NH_2$, —NH—$COOC_2H_5$ oder

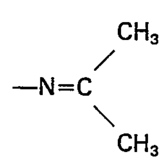

steht,

n den Wert 1 oder 2 hat, und

(a) wenn Z für —O— steht,

$R^1$ für ein Wasserstoffatom, eine $C_1$—$C_5$-Alkylgruppe, substituiert durch eine $C_1$—$C_5$-Alkoxy-, $C_1$—$C_5$-Alkylthio-, $C_2$—$C_5$-Alkanoylamino-, Allyloxy- oder Tetrahydrofurfuryloxygruppe, eine $C_1$—$C_5$-Alkoxygruppe, substituiert durch eine 2-Furyl-, Phenyl- oder $C_1$—$C_5$-Alkoxygruppe, steht,

$R^2$ und $R^3$ unabhängig voneinander für ein Wasserstoffatom oder eine $C_1$—$C_5$-Alkylgruppe, substituiert durch eine $C_1$—$C_5$-Alkoxygruppe, stehen,

mit der Maßgabe, daß $R^1$, $R^2$ und $R^3$ nicht gleichzeitig Wasserstoffatome sind und daß, wenn $R^3$ für eine $C_1$—$C_5$-Alkylgruppe, die durch eine $C_1$—$C_5$-Alkoxygruppe substituiert ist, steht, $R^1$ ein Halogenatom sein kann, und

$R^4$ und $R^5$ ein Wasserstoffatom darstellen und

(b) wenn Z für —S— oder

—N—
|
$R^8$

steht,

$R^1$ für eine Methoxyethyl- oder $C_1$—$C_5$-Alkylgruppe oder ein Halogenatom steht,

$R^2$ und $R^4$ unabhängig voneinander für ein Halogenatom oder eine Methylgruppe stehen, und

$R^3$ und $R^5$ ein Wasserstoffatom darstellen,

oder deren Salz, dadurch gekennzeichnet, daß man eine Verbindung der Formel

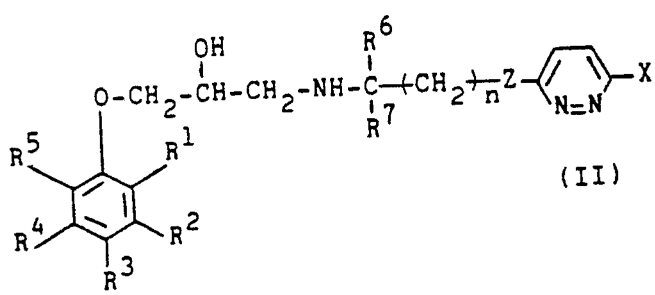

(II)

worin X für ein Halogenatom steht und $R^1$, $R^2$, $R^3$, $R^4$, $R^5$, $R^6$, $R^7$, Z und n wie oben definiert sind, mit (a) Hydrazin oder Hydrazinhydrat, wenn Z in Formel (II) für —O— oder —S— steht, oder (b) Ethylcarbazinat, wenn Z in Formel (II) für

—N—
|
$R^8$

steht, umsetzt, sodann erforderlichenfalls die resultierende Verbindung, wenn sie Formel (I), worin Y für —NH—$COOC_2H_5$ steht, entspricht, hydrolysiert und erforderlichenfalls die resultierende Verbindung, wenn sie der Formel (I), bei der Y für —$NH_2$ steht, entspricht, mit Aceton umsetzt und weiterhin erforderlichenfalls die Verbindung der Formel (I) in ihr Salz umwandelt.

**Revendications pour les Etats contractants: BE CH DE FR GB IT LI LU NL SE**

1. Un composé d'hydrazinopyridazine représenté par la formule

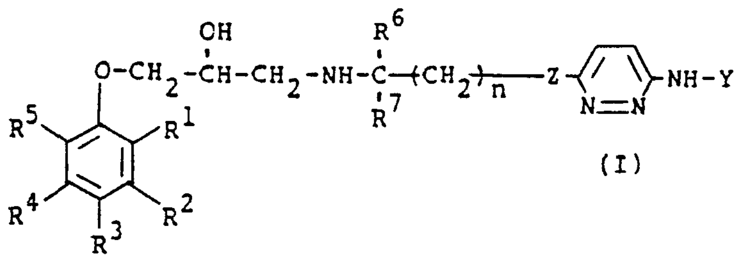

(I)

dans laquelle

$R^6$ et $R^7$, indépendamment l'un de l'autre, représentent un atome l'hydrogène ou un groupe méthyle;

Z représente —O—, —S— ou

—N—
|
$R^8$

où $R^8$ représente un atome d'hydrogène ou un groupe alkyle en $C_1$—$C_5$;

Y représente —$NH_2$, —NH—$COOC_2H_5$ ou

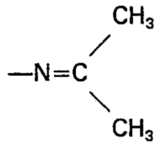 ;

n est 1 ou 2; et

(a) si Z représente —O—,

$R^1$ représente un atome d'hydrogène, un groupe alkyle en $C_1$—$C_5$ substitué par un groupe alcoxy en $C_1$—$C_5$, alkylthio en $C_1$—$C_5$, alcanoylamino en $C_2$—$C_5$, allyloxy ou tétrahydrofurfuryloxy, un groupe alcoxy en $C_1$—$C_5$ substitué par un groupe 2-furyle, phényle ou alcoxy en $C_1$—$C_5$,

$R^2$ et $R^3$, indépendamment l'un de l'autre, représentent un atome d'hydrogène ou un groupe alkyle en $C_1$—$C_5$ substitué par un groupe alcoxy en $C_1$—$C_5$,

avec la condition que $R^1$, $R^2$ et $R^3$ ne soient pas simultanément des atomes d'hydrogène, et que si $R^3$ représente un groupe alkyle en $C_1$—$C_5$ substitué par un groupe alcoxy en $C_1$—$C_5$, $R^1$ puisse représenter un atome d'halogène, et

$R^4$ et $R^5$ représentent un atome d'hydrogène; et

(b) si Z représente —S— ou

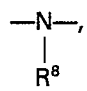

$R^1$ représente un groupe méthoxyéthyle ou alkyle en $C_1$—$C_5$ ou un atome d'halogène,

$R^2$ et $R^4$, indépendamment l'un de l'autre, représentent un atome d'halogène ou un groupe méthyle, et $R^3$ et $R^5$ représentent un atome d'hydrogène, ou un sel de ce composé.

2. Le composé de la revendication 1, dans lequel $R^6$ et $R^7$ sont chacun un groupe méthyle.

3. Le composé de la revendication 1 ou 2 à titre de substance pharmaceutiquement active.

4. Le composé de la revendication 1 ou 2 à titre d'agent antihypertensif.

5. Un procédé pour produire le composé répondant à la formule (I) donnée dans la revendication 1, qui consiste à faire réagir un composé représenté par la formule

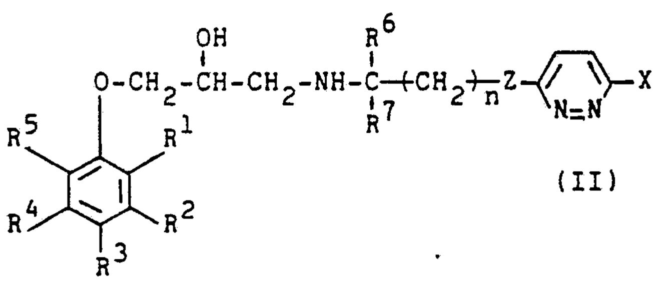

(II)

dans laquelle X représente un atome d'halogène et $R^1$, $R^2$, $R^3$, $R^4$, $R^5$, $R^6$, $R^7$, Z et n sont comme définis dans la revendication 1, avec (a) l'hydrazine ou l'hydrate d'hydrazine lorsque Z représente —O— ou —S— dans la formule (II), ou (b) le carbazinate d'éthyle lorsque Z représente

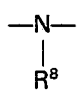

dans la formule (II); puis, au besoin, à hydrolyser le composé résultant lorsqu'il correspond à la formule (I) où Y est —NH—$COOC_2H_5$; et, au besoin, à faire réagir le composé résultant avec l'acétone lorsqu'il correspond à la formule (I) où Y est —$NH_2$; et, au besoin encore, à transformer le composé de formule (I) en un de ses sels.

6. Un médicament contenant un composé répondant à la formule (I) donnée dans la revendication 1 ou un sel pharmaceutiquement acceptable de ce composé et un support ou diluant pharmaceutiquement acceptable.

**Revendication pour l'Etat contractant: AT**

Un procédé pour produire un composé d'hydrazinopyridazine représenté par la formule

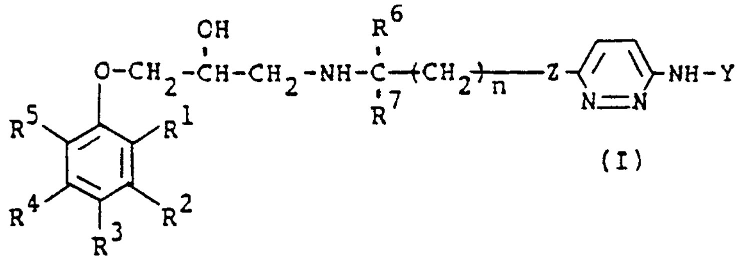

(I)

dans laquelle

$R^6$ et $R^7$, indépendamment l'un de l'autre, représentent un atome d'hydrogène ou un groupe méthyle;

Z représente —O—, —S— ou

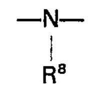

où $R^8$ représente un atome d'hydrogène ou un groupe alkyle en $C_1$—$C_5$;

Y représente $—NH_2$, $—NH—COOC_2H_5$ ou

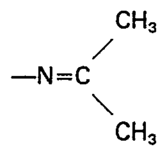

;

n est 1 ou 2; et

(a) si Z représente —O—,

$R^1$ représente un atome d'hydrogène, un groupe alkyle en $C_1$—$C_5$ substitué par un groupe alcoxy en $C_1$—$C_5$, alkylthio en $C_1$—$C_5$, alcanoylamino en $C_2$—$C_5$, allyloxy ou tétrahydrofurfuryloxy, un groupe alcoxy en $C_1$—$C_5$ substitué par un groupe 2-furyle, phényle ou alcoxy en $C_1$—$C_5$,

$R^2$ et $R^3$, indépendamment l'un de l'autre, représentent un atome d'hydrogène ou un groupe alkyle en $C_1$—$C_5$ substitué par un groupe alcoxy en $C_1$—$C_5$,

avec la condition que $R^1$, $R^2$ et $R^3$ ne soient pas simultanément des atomes d'hydrogène et que si $R^3$ représente un groupe alkyle en $C_1$—$C_5$ substitué par un groupe alcoxy en $C_1$—$C_5$, $R^1$ puisse représenter un atome d'halogène, et

$R^4$ et $R^5$ représentent un atome d'hydrogène; et

(b) si Z représente —S— ou

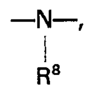

$R^1$ représente un groupe méthoxyéthyle ou alkyle en $C_1$—$C_5$ ou un atome d'halogène,

$R^2$ et $R^4$, indépendamment l'un de l'autre, représentent un atome d'halogène ou un groupe méthyle,

$R^3$ et $R^5$ représentent un atome d'hydrogène, ou un sel de ce composé, qui consiste à faire réagir un composé représenté par la formule

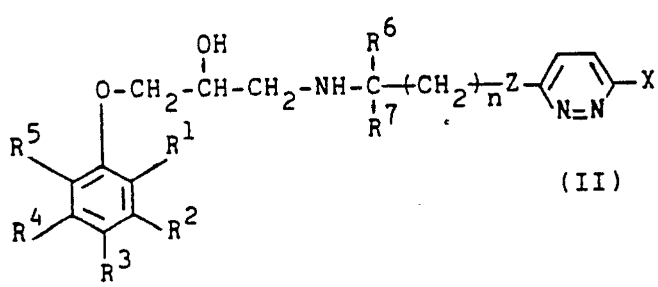

(II)

dans laquelle X représente un atome d'halogène et $R^1$, $R^2$, $R^3$, $R^4$, $R^5$, $R^6$, $R^7$, Z et n sont comme définis ci-dessus,

avec (a) l'hydrazine ou l'hydrate d'hydrazine lorsque Z représente —O— ou —S— dans la formule (II), ou (b) le carbazinate d'éthyle lorsque Z représente

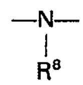

dans la formule (II); puis, au besoin, à hydrolyser le composé résultant lorsqu'il correspond à la formule (I) où Y est —NH—$COOC_2H_5$; et, au besoin, à faire réagir le composé résultant avec l'acétone lorsqu'il correspond à la formule (I) où Y est —$NH_2$; et, au besoin encore, à transformer le composé de formule (I) en un de ses sels.